# EUROPEAN PATENT SPECIFICATION

(11) **EP 3 066 468 B1**
(45) Date of publication and mention of the grant of the patent: **29.03.2023**
(21) Application number: 14799379.4
(22) Date of filing: 07.11.2014
(51) Int. Cl.: G01N 33/50, A61K 31/00, A61K 31/573

(54) **SCREENING ASSAY FOR AGENTS THAT INFLUENCE BETA CELL NUMBER AND/OR PHENOTYPE**
SCREENINGTEST FÜR WIRKSTOFFE ZUR BEEINFLUSSUNG DER ANZAHL UND/ODER DES PHÄNOTYPS VON BETAZELLEN
TEST DE CRIBLAGE D'AGENTS QUI INFLUENCENT LE NOMBRE DE CELLULES BÊTA ET/OU LE PHÉNOTYPE

(30) Priority: 07.11.2013 EP 13191960
(43) Date of publication of application: 14.09.2016
(73) Proprietor: Vrije Universiteit Brussel, 1050 Brussel (BE); OPUS NV, 1761 Borchtlombeek (BE)
(72) Inventor: ASSEFA, Zerihun, 1090 Jette (BE); STANGE, Geert, 1090 Jette (BE); PIPELEERS, Daniel, 1090 Jette (BE); HELLEMANS, Karine, 1090 Jette (BE)
(74) Representative: Arnold & Siedsma
(86) International application number: PCT/EP2014/074021
(87) International publication number: WO 2015/067744

(56) References cited:
- EP-A1- 1 297 841
- WO-A2-2013/049375
- US-A1- 2009 149 435
- M. KOIZUMI ET AL: "Sub-chronic stimulation of glucocorticoid receptor impairs and mineralocorticoid receptor protects cytosolic Ca2+ responses to glucose in pancreatic -cells", JOURNAL OF ENDOCRINOLOGY, vol. 197, no. 2, 1 May 2008 (2008-05-01), pages 221-229, XP055101901, ISSN: 0022-0795, DOI: 10.1677/JOE-07-0462
- G. A. MARTENS ET AL: "Functional characteristics of neonatal rat cells with distinct markers", JOURNAL OF MOLECULAR ENDOCRINOLOGY, vol. 52, no. 1, 18 September 2013 (2013-09-18), pages 11-28, XP055163735, ISSN: 0952-5041, DOI: 10.1530/JME-13-0106
- RATMAN DARIUSZ ET AL: "How glucocorticoid receptors modulate the activity of other transcription factors: A scope beyond tethering", MOLECULAR AND CELLULAR ENDOCRINOLOGY, vol. 380, no. 1, 23 December 2012 (2012-12-23), pages 41-54, XP028728171, ISSN: 0303-7207, DOI: 10.1016/J.MCE.2012.12.014

## Description

### FIELD OF THE INVENTION

The present invention in general relates to a method for identifying compounds that influence beta cell number and/or phenotype, more in particular beta cell differentiation, proliferation and/or expansion. Said method comprising culturing beta cells or precursors thereof in medium comprising glucose, and determining the ability of said compound to induce the glucocorticoid receptor dependent-transactivation pathway in said beta cells.

### BACKGROUND TO THE INVENTION

Reduced beta cell mass is a hallmark of type 1 diabetes and a clinically aggravating factor in type 2 diabetes. It therefore became the target of projects searching for agents that can increase the number of beta cells in patients. Although several in vivo and in vitro conditions have been reported to induce beta cell proliferation (Heit et al., 2006; Nielsen et al., 1999), their implementation to increase beta cell mass in diabetic models has been limited and variable. There are, so far, virtually no published data on compounds that increase the absolute number of primary beta cells in vitro. In order to identify compounds with such effect, we have now developed a method for determining and following the number of living beta cells and/or for assessing beta cell phenotype during two weeks of culture. During this period, influences on beta cell survival can for example be analyzed by vital staining, those on beta cell proliferative activity by thymidine-analog incorporation and cell number counts. The effect of glucose can be examined in adolescent cells at concentrations that were previously found to recruit beta cells into metabolic and biosynthetic activity (Kiekens et al., 1992). Since beta cells with higher glucose sensitivity exhibited a higher glucokinase activity (Heimberg et al., 1993), we investigated whether a glucokinase activator helps recruit beta cells into proliferation. Our in vitro assay can thus provide direct support for this mechanism and localize the responsive cells within the functional heterogeneity of the beta cell population (Pipeleers et al., 1994). Patent document WO2013049375 discloses that agents promoting PDGFR signaling promote beta cell proliferation.

In particular the culture method of the present invention encompasses culturing primary mammalian beta cells or precursors thereof in medium comprising glucose; more in particular at a concentration of about 1 - about 50 mM. The presence of glucose in the medium, in particular at elevated levels i.e. 10-20 mM was found to consistently increase the number of beta cells from adolescent rats *in vitro.* Hence the method according to this invention is a novel useful tool for analyzing compounds for their effect on beta cell numbers and/or phenotype over time, and their potential in the treatment of e.g. diabetes mellitus.

Using this method, we have surprisingly found that glucocorticoid receptor transactivation stimulates adult beta cells to recapitulate a proliferative phenotype. Hence, we present herein a method for identifying compounds that stimulate beta cell differentiation, proliferation and/or expansion, based on their ability to induce the glucocorticoid receptor dependent-transactivation pathway in said beta cells.

### SUMMARY OF THE INVENTION

The invention is set out in the appended set of claims.

In a first aspect the present invention provides a method for identifying a compound that simulates beta cell differentiation, proliferation and/or expansion, by determining the ability of said compound to induce the glucocorticoid receptor dependent-transactivation pathway in said beta cells, wherein a compound that is capable of inducing the glucocorticoid receptor-dependent transactivation pathway, is identified as a compound that stimulates beta cell differentiation, proliferation and/or expansion.

Said method is herein after referred to as the 'transactivation assay'.

In a further aspect, testing the ability of said compound to induce the glucocorticoid receptor dependent-transactivation pathway in said beta cells comprises the steps of:
- culturing beta cells or precursors thereof in the absence (control sample), or presence (test sample) of a test compound,
- measuring the expression of one or more transactivation markers in said beta cells or precursors thereof in both samples;
wherein a compound that increases expression of said one or more transactivation markers in said test sample in comparison with said control sample, is identified as a compound that stimulates beta cell differentiation, proliferation and/or expansion.

Specifically, in the context of the present invention, the transactivation markers may be selected from the list comprising: FKBP5, MKP1, IL-1 Ra, Fgb and Fgg. The present application in particular provides transactivation markers selected from the list comprising: FKBP5, MKP1 and IL-1 Ra.

The method as defined above, may further comprise measuring the expression of one or more transrepression markers in said beta cells;
wherein a compound that increases expression of one or more transactivation markers, and does not decreases the expression of one or more transrepression markers in said test sample in comparison with said control sample; is identified as a compound that stimulates beta cell differentiation, proliferation and/or expansion.

Specifically, in the context of the present invention, the transrepression markers may be selected from the list comprising: ICAM1, CXCL11, Nfkbie, Myd88, Birc3. The present application in particular provides transrepression markers selected from the list comprising: ICAM1 and CXCL11

The present invention further provides a method for identifying a compound that stimulates beta cell differentiation over time, said method comprising the steps of:
- providing a compound that is capable of inducing the glucocorticoid receptor dependent-transactivation pathway in beta cells or precursors thereof
- culturing beta cells or precursors thereof in the presence of said compound
- measuring over time in said beta cells the expression of one or more differentiation markers, selected from MafB, DLK1, NPY, NNAT and TRH; and one or more maturation markers selected from MafA and PDX1 ;
wherein a compound that increases expression of at least one of said differentiation markers and does not decrease the expression of said one or more maturation markers, over time, is identified as a compound that stimulates beta cell differentiation

Said method is herein after referred to as the 'differentiation assay'.

The present invention also provides a method for identifying a compound that stimulates beta cell proliferation over time, said method comprising the steps of:
- providing a compound that is capable of inducing the glucocorticoid receptor dependent-transactivation pathway in beta cells or precursors thereof
- culturing beta cells or precursors thereof in the presence of said compound and a thymidine-analog
- measuring the degree of incorporation of said thymidine-analog in said beta cells over time;
wherein a compound that increases thymidine incorporation in said beta cells over time, is identified as a compound that influences beta cell proliferation

Said method is herein after referred to as the 'proliferation assay'.

In a further aspect, the present invention provides a method for identifying a compound that stimulates beta cell expansion over time, said method comprising the steps of:
- providing a compound that is capable of inducing the glucocorticoid receptor dependent-transactivation pathway in beta cells or precursors thereof
- culturing beta cells or precursors thereof in the presence of said compound
- measuring the absolute number of living beta cells over time using high content imaging,
wherein a compound that increases the absolute number of living beta cells over time is identified as a compound that stimulates beta cell expansion

Said method is herein after referred to as the 'expansion assay'.

In particular in the context of the present invention, the compound that is capable of inducing the glucocorticoid receptor dependent-transactivation pathway, is identified using the 'transactivation assay' as defined herein above.

In a preferred embodiment, the present invention thus provides a method for identifying a compound suitable for the treatment of diabetes mellitus; said method comprising the steps of
- performing the 'transactivation assay' as provided herein,
- performing the 'differentiation assay' as provided herein,
- performing the 'proliferation assay' as provided herein, and
- performing the 'expansion assay' as provided herein
wherein a compound that is capable of inducing the glucocorticoid receptor dependent-transactivation pathway in beta cells or precursors thereof as determined using the 'transactivation assay', is capable of stimulating beta cell differentiation as determined using the 'differentiation assay', is capable of stimulating beta cell proliferation as determined using the 'proliferation assay', and is capable of stimulating beta cell expansion using the 'expansion assay'; is identified as a compound that is suitable for the treatment of pathologies characterized by a change in beta cell differentiation, proliferation and/or expansion, such as for example diabetes mellitus.

Preferably, the beta cells or precursors thereof according to the present application, are cultured in Ham F10 medium supplemented with about 1 - about 50 mM glucose, about 0 - about 50 µM IBMX (isobutyl-1-methylxanthine), about 0.1% - about 4% albumax I, about 0.1 mg/ml streptomycin, and about 0.075 mg/ml penicillin.

More in particular, the beta cells or precursors thereof, are preferably cultured on extracellular matrices, such as for example 804G matrices.

In the context of the present application, the measuring over time may be performed after about 1-15 days of culture, in particular after about 1, about 3, about 6, about 9, about 12 or about 15 days of culture.

The beta cells or precursors thereof, in accordance with the present application, are preferably isolated from a perinatal mammal, adolescent mammal, or adult mammal; more in particular, from human, porcine, rat, and mouse.

The present application further provides a method for producing a pharmaceutical composition comprising combining a compound that is capable of inducing the glucocorticoid dependent-transactivation pathway, with a pharmaceutically acceptable carrier.

The present application also provides a method for producing a pharmaceutical composition for the treatment of diabetes mellitus, comprising combining a compound that is capable of inducing the glucocorticoid dependent-transactivation pathway, with a pharmaceutically acceptable carrier.

In particular, in said methods, the compound that is capable of inducing the glucocorticoid dependent-transactivation pathway, is identified using the 'transactivation assay' as defined herein.

### BRIEF DESCRIPTION OF THE DRAWINGS

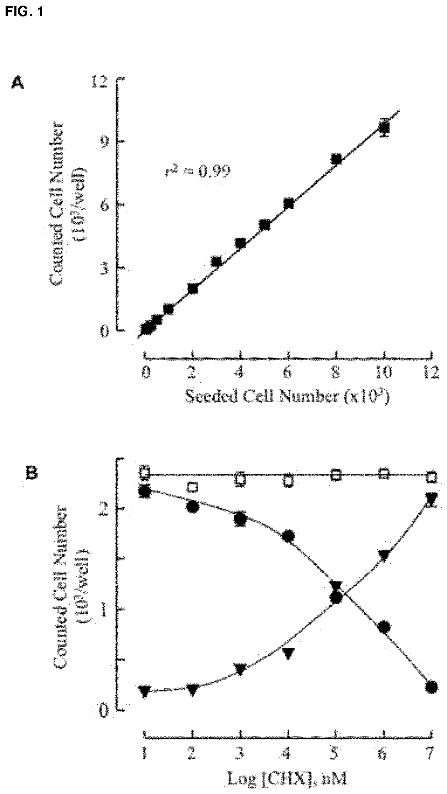

**FIGURE 1****: Image analysis for counting the number of living and dead beta cells per well and determining the percentages of EdU-positive cells.** Purified rat beta cells were seeded in 96- or 384-well plates and stained by Hoechst 33342 (Ho) and propidium iodide. Whole-well images were taken at a resolution that allows identification and counting of all cells distinguishing PI-positive and PI-negative cells (a). IPLab and AttoVision software packages were used for image processing and quantification. Following image segmentation, every cell is assigned a number of living (Ho-pos, PI-neg) and dead cells (Ho-pos-PI-pos) are determined. Induction of apoptosis by cycloheximide-CHX causes within 48h a dose-dependent increase in the number of dead cells (black triangles) and decrease in the number of living cells (black circles) without change in the total numbers (while squares) that are counted (b) (means ± SEM of three independent experiments). Image segmentation delineates nuclear and cytoplasmic boundaries of every cell through dynamic thresholding that detects changes in Hoechst fluorescence above background; the cytoplasm was designated by the area between the nuclear boundary and a three pixel-wide annular ring drawn around it (not shown). Following staining for insulin and EdU, the nuclear image is superimposed on the EdU and insulin immunofluorescence images and fluorescence intensity is averaged for pixels within the nuclear or cytoplasmic area so that the percentages of single or double-positive cells can be determined.

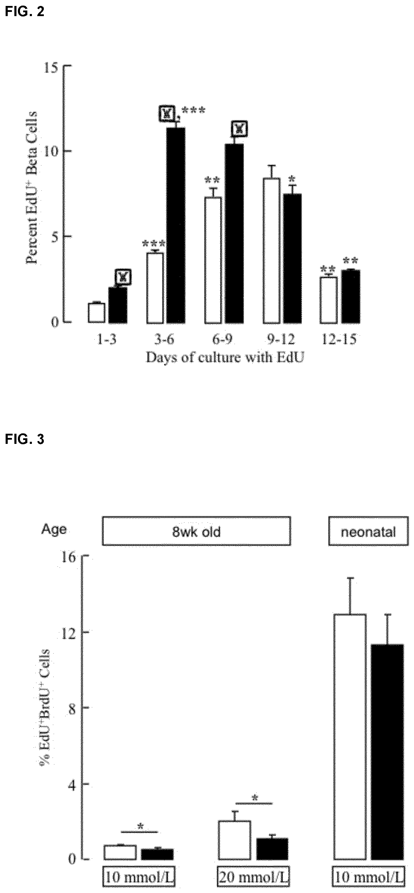

**FIGURE 2****: Effect of glucose on percent beta cells in DNA synthesis.** Beta cells from 8-week old rats were cultured for the indicated periods at 10 mmol/L (white bars) or 20 mmol/L (black bars) glucose in presence of EdU. At the end of each period, the percent EdU⁺Insulin⁺ cells is determined. Data represent means ± SEM; ≠, p<0.05; #, p<0.001 10 mmol/L versus 20 mmol/L; *, p<0.05; **, p<0.01; ***, p<0.001 versus preceding period; n=3.
**FIGURE 3****: Adult and neonatal rat beta cells differ in their post-mitotic refractory period.** Comparison of the predicted (white bars) and counted (black bars) percentages of EdU⁺BrdU⁺ beta cells from adult and neonatal rats, by assuming stochastic recruitment of beta cells into the cell cycle. Cells were cultured at indicated glucose concentration and labeled with EdU and BrdU as described in Methods. Data are expressed as means ± SEM; *, p< 0.05, n=3-5.

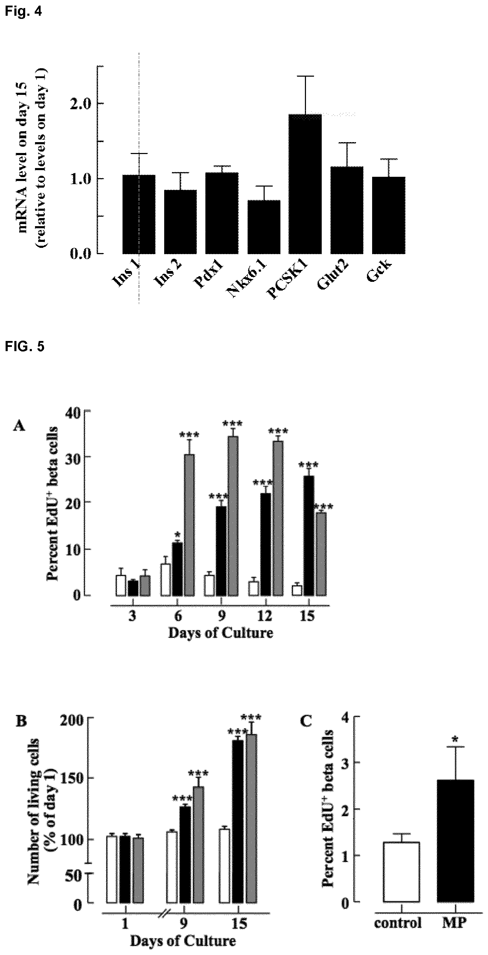

**FIGURE 4****: Stability in beta-cell characteristic genes following glucose-induced recruitment of young adult beta cells into proliferative activity.** Beta cells purified from 8 week-old rats were cultured for 15 days at 10 mmol/l glucose. The mRNA levels of Ins1, Ins 2, Pdx1, Nkx6.1, PCSK1, Glut2 and Gck were quantified by qPCR and expressed relative to the levels in freshly purified cells (means ans rified cells (means ns eta cells into proliferative activity.n
**FIGURE 5****. Glucocorticoids increase the number of adult rat beta cells by time-dependent activation of proliferation. (A)** Purified cells were cultured in control (white bars), in HC (black bars) or MP (grey bars) containing medium. The percent EdU⁺ cells were determined on the indicated days after adding the analog during the preceding 72h of culture. The data are presented as means ± SEM; n=3-4; *, p<0.05; ***, p<0.001 versus control. **(B)** Both HC and MP treatments can increase the absolute number of living beta cells in culture. Cells were cultured in control (white bars), HC (black bars) or MP (grey bars) containing medium and the number of living cells was determined on the indicated days. Values were adjusted as percentage of the number of cells on day 1 and represent the means ± SEM; ***, p<0.001 versus day 1 numbers (HC, n=30; MP, n=8). **(C)** Isolated human beta cells were cultured with or without 1µM MP for a total of 9-days, and EdU was added during the last 3-days of culture. Percent EdU⁺ beta cells is shown as mean ± SEM; *, p<0.05 versus control (n=9).

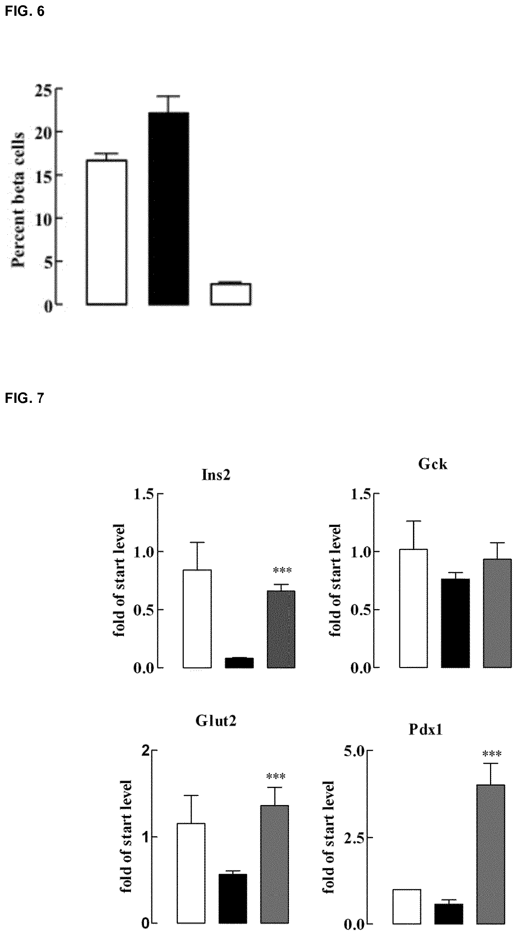

**FIGURE 6****. Time-course of GC-induced DNA synthesis in rat beta cells.** HC-induced recruitment of beta cells into DNA synthesis and cell cycle re-entry of previously recruited cells. Adult rat beta cells were cultured at 10mM glucose in the presence of HC for 10 days and labeled sequentially with EdU (day 3 to 6) and BrdU (day 7 to 10). The percentages of EdU⁺BrdU⁻ (white bar), BrdU⁺EdU⁻(black bar) and EdU⁺BrdU⁺ (hatched bar) on day 10 are shown as means ± SEM; n=5.
**FIGURE 7****: HC withdrawal from culture medium restores the expression of a number of defining markers for mature beta cells.** Purified beta cells were cultured in control medium for 15 day (white bars), HC-containing medium for 15 days (black bars) or HC for 9 days and control medium for 6 days (grey bars). The level of mRNA for the indicated genes was quantified by qPCR. Data are expressed as fold of levels in freshly purified beta cells and represent means ± SEM; compared to continuous culture with HC: ***, p<0.001; n=4.

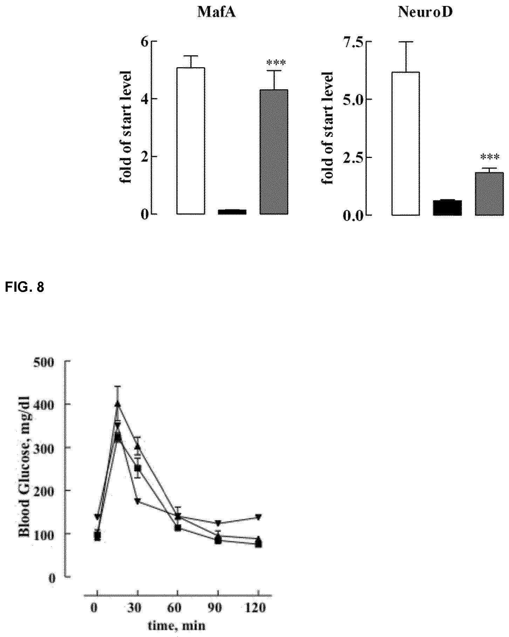

**FIGURE 8****. Alloxan-induced diabetes in mice can be reversed with implantation of in vitro-generated beta cells.** Sorted beta cells were cultured in control medium or in the presence of HC up to day 9 and then switched to basal condition for the last six days of culture. IPGTT was used to determine glucose clearance rate. Glucose was administered (3g/kg body weight) by in intraperitoneal injection into a 2h-fasted animals and blood glucose levels were measured at the indicated time points during the following 120-min. Data are from animals transplanted with control cells (black triangles; n=3), in vitro expanded cells (black squares; n=5) and normal control animals (inverted triangles; n=2).

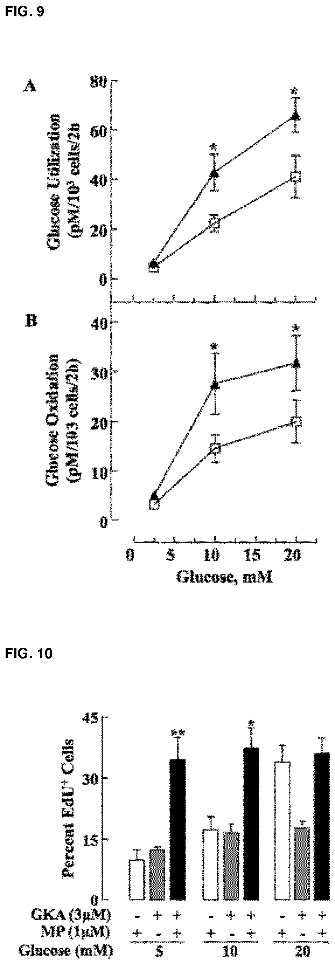

**FIGURE 9****: Glucose utilization and oxidation by isolated beta cells after treatment with HC.** Purified rat beta cells were cultured for six days in control medium (white squares) or with HC (black triangles) at 10mM glucose. Glucose utilization **(A)** was determined by the rate of D-(5-³H)glucose conversion into ³H₂O and glucose oxidation **(B)** by that of D-(U-¹⁴C)glucose into ¹⁴CO₂ during a 2h incubation period at the indicated concentrations of glucose without HC. Values represent means ± SEM, n=6; *, p<0.05 versus control.
**FIGURE 10****: Effect of glucokinase activator (GKA) on HC-induced DNA synthesis in beta cells.** Rat beta cells were cultured in 5, 10 or 20mM glucose for six days with MP and GKA, separately or in combination. EdU was added on day 3 and the percent EdU⁺ cells determined on day 6. Means ± SEM of 4 independent experiments are shown; *, p<0.05; **, p<0.01 versus corresponding condition without GKA.

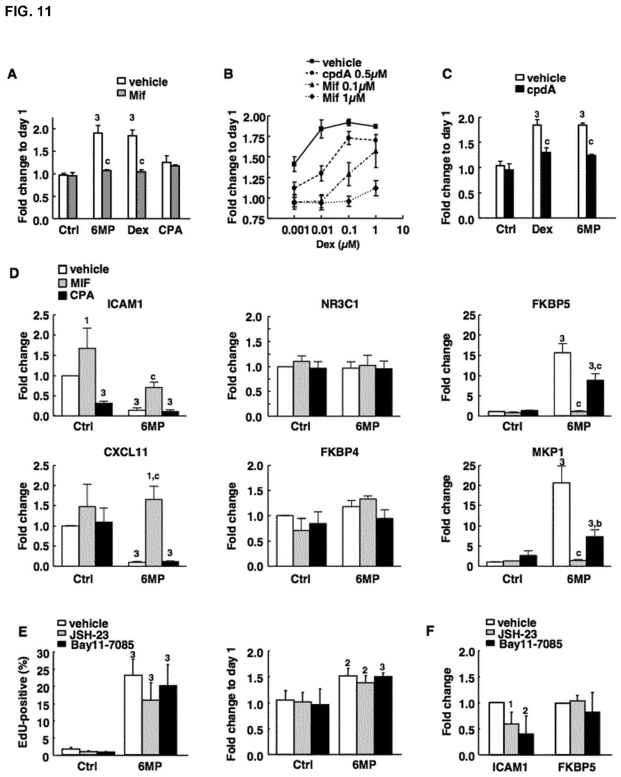

**FIGURE 11****: Glucocorticoid receptor transactivation supports beta cell expansion.** To determine the contribution of GR in glucocorticoid-stimulated beta cell proliferation, and to evaluate whether transrepression or transactivation mechanisms are involved, beta cells were exposed for 15 days to glucocorticoids (6MP, DEX), the segregated agonist compound A (CPA 0.5 µM) w/wo inhibitor mifepristone (MIF 1µM). CPA supports transrepression, but does not induce receptor dimerization thereby preventing classical GR transactivation. **A)** Between day 1 and 15 cell numbers nearly double in the presence of 6MP (1µM) and DEX (0.01 µM), an effect inhibited by MIF (1µM). CPA (0.5 µM) showed no change of beta cell numbers over 15 days. **B**) Dose response curve showing the competitive interaction of MIF and CPA with DEXstimulated increase of beta cell numbers between d1 and 15. CPA (0.5µM) showed a maximal inhibition of the increase of cell numbers in combination with 0.01 µM DEX, whereas at this concentration beta cell expansion was prevented by 0.1 µM MIF. **C)** CPA (0.5µM) showed a comparable inhibitory effect on d15/d1 gain in cell numbers as observed for 0.01 µM DEX when combined with 1µM 6MP which is consistent with the lower binding potential of 6MP to GR. **D)** The segregated action of CPA on GR was further documented by Q-PCR, evaluating the changes in mRNA levels of typical transrepression (Icam1, CXCL11) and transactivation targets (FBPB5, MKP1) and non-responsive genes (NR3C1, FKBP4) after 9 days exposure to MIF (1 µM, gray bars), or CPA (0.5 µM, black bars) under basal culture conditions (Ctrl), or in presence of 6MP (1 µM) (white bars). **E)** Inhibition of NFkB using JSH-23 (10 µM) or Bay11-7085 (5 µM) does not inhibit 6MP induced DNA-synthesis and increase in cell numbers. **F)** Q-PCR showing the effect of NFkB-inhibitors on ICAM1 and FKBP5 mRNA levels relative to vehicle control. N = 3 to 4, data show mean ± se. ¹ p < 0.05, ² p < 0.01, ³ p < 0.001 compared to Ctrl; ^{a} p < 0.05, ^{b} p < 0.01, ^{c} p < 0.001 as compared to glucocorticoids (DEX or 6MP).

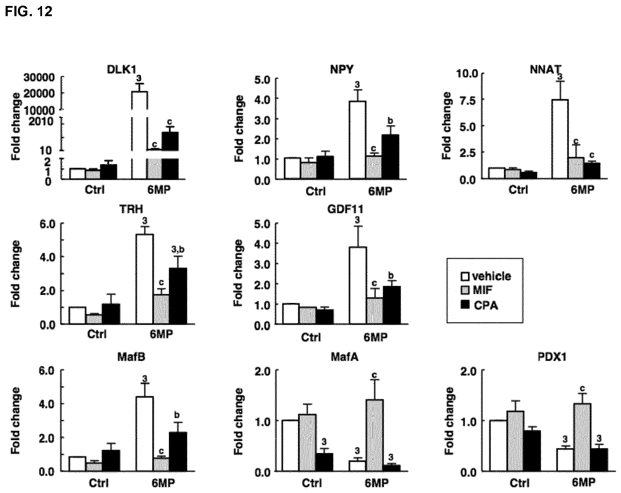

**FIGURE 12****: GR transrepression and transactivation mechanisms determine the phenotype of glucocorticoid-activated beta cells.** In order to describe the impact of GR-activation on the beta cell phenotype, and in order to evaluate whether trans-repression or transactivation mechanisms are involved, we analyzed the mRNA levels of a selection of glucocorticoid responsive genes after 9 days culture in the absence or presence of 6MP (white bars) w/wo MIF (grey bars) or CPA (black bars). Phenotype markers were selected on basis of their putative role under specific growth-conditions such as embryogenesis and beta cell differentiation (MafA, Pdx1, GDF11), the neonatal phase (DLK1, NPY, NNAT) and pregnancy (MafB, TRH). The expression levels were normalized to 4 housekeeping genes (β-actin, HPRT1, rplp2 and psmc5) and expressed relative to the basal control condition. N = 4-5, data show mean ± se. ¹ p < 0.05, ² p < 0.01, ³ p < 0.001 compared to control; ^{a} p < 0.05, ^{b} p < 0.01, ^{c} p < 0.001 as compared to 6MP.

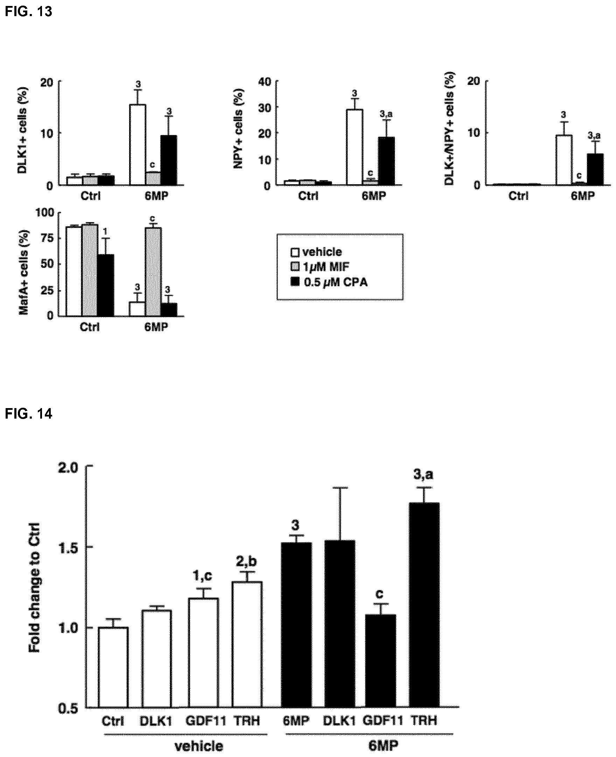

**FIGURE 13****: Quantification of marker+ beta cells, effect of CPA a segregated GR modulator.** Beta cells were cultured for 9 days in the absence or presence of 6MP (white bars) w/wo MIF (grey bars) or CPA (black bars) and used for immunochemistry. Marker+ cells were quantified using a pathway bioimager and attovision-software as described under M&M. N = 4, data show mean ± se. ¹ p < 0.05, ² p < 0.01, ³ p < 0.001 compared to control; ^{a} p < 0.05, ^{b} p < 0.01, ^{c} p < 0.001 as compared to 6MP.
**FIGURE 14****: Glucocorticoid-stimulation of beta cell proliferation does not require paracrine signals**
   In order to explore the potential paracrine effect of DLK1, NPY, GDF11 and TRH on beta cell proliferation beta cells were cultured w/wo these factors. Beta cells were cultured for 15 days in basal control media or 6MP (1 µM), w/wo DLK1 (0.5 µM), GDF11 (10 nM) and TRH (5 nM). The results are expressed relative to the basal control condition. N = 3-4, data show mean ± se. ¹ p < 0.05, ² p < 0.01, ³ p < 0.001 compared to control; ^{a} p < 0.05, ^{b} p < 0.01, ^{c} p < 0.001 as compared to 6MP.

### DETAILED DESCRIPTION OF THE INVENTION

The present invention is based on the finding that a culture of (primary) beta cells in a (serum-free) medium comprising glucose can be used for the parallel assessment of beta cell number and/or phenotype; in particular beta cell viability, cell numbers, proliferation, and/or function (e.g. based on insulin synthesis), with the detection of short and long-term effects on each read out (see examples). Using this novel culture method, we have surprisingly found that glucocorticoid receptor transactivation stimulates adult beta cells to recapitulate a proliferative phenotype.

It is accordingly a first aspect of the present invention, to provide a method for identifying compounds that influence beta cell number and/or phenotype, said method comprising; culturing beta cells or precursors thereof in medium comprising glucose.

In the method according to the present application, the beta cells or precursors thereof are cultured in serum-free medium comprising glucose; more in particular in Ham F10 medium supplemented with about 1 - about 50 mM glucose, about 0 - about 50 µM IBMX (isobutyl-1-methylxanthine), about 0.1% - about 4% albumax I, about 0.1 mg/ml streptomycin, and about 0.075 mg/ml penicillin.

In a first embodiment, said method comprises: culturing beta cells or precursors thereof in medium comprising glucose in the presence or absence of the compound(s) to be tested, and comparing beta cell number and/or phenotype over time in both conditions, in order to assess the effect of said compound(s).

In a specific embodiment, in the step of comparing beta cell number and/or phenotype; a relative change in beta cell number and/or phenotype in the presence of said compound(s) when compared to the beta cell number and/or phenotype in the absence of said compound(s), is indicative for the ability of said compound(s) to influence beta cell number and/or phenotype.

Within said screening methods, assessment of beta cell phenotype is based on one or more parameters selected from: state of differentiation; state of structural integrity; state of proliferative activity; viability; or state of functional activity such as insulin biosynthesis, insulin secretion, or glucose responsiveness.

In a particular embodiment of the present invention, the methods of the present invention allow to screen for compounds that enhance functional beta cell mass (numbers). Within said embodiment beta cell activity is evaluated by determining changes in absolute beta cell numbers optionally in combination with determining beta cell phenotype (in particular insulin synthesis). It is accordingly an object of the present invention to provide a method for identifying compounds that influence beta cell number and/or phenotype, said method comprising culturing beta cells or precursors thereof in (serum-free) medium comprising glucose in the presence or absence of the compound to be tested, comparing beta cell number and/or phenotype over time in both conditions; and wherein a relative change in beta cell number and/or phenotype in the presence of said compound when compared to the beta cell number and/or phenotype in the absence of said compound, is indicative for the ability of said compound to influence beta cell number and/or phenotype. Said beta cell phenotype may be evaluated by determining changes in state of differentiation; state of structural integrity; state of proliferative activity; viability; or state of functional activity such as insulin biosynthesis, insulin secretion, or glucose responsiveness.

As evident from the foregoing embodiments, the assessment of beta cell phenotype may be based on the determination of insulin synthesis comprising determining insulin content in the cells and/or in the medium.

As will become apparent from the examples hereinafter, the beta cells or precursors thereof, as used in the screening method of the present invention may be at different stages of differentiation. In a particular embodiment said beta cells or precursors thereof may be isolated from a perinatal, adolescent or adult mammal. Evidently, the methods of the present invention are not restricted to particular mammalian cells, but in a particular embodiment the mammal may be selected from the list comprising human, porcine, rat, and mouse.

In the context of the present invention, the term `perinatal' is to be understood as the period immediately before and after birth, and includes the neonatal stage (newborn mammals). For example, when referring to rats, perinatal rats are meant to include rats from about 5 days before, until about 3 days after birth; neonatal rats are meant to include rats of about 2-3 days after birth; when referring to humans, perinatal is referred to as the period between 5 months before and about one month after birth.

In the context of the present invention, the term 'adolescent' is meant to be the phase during a mammals life between youth and adulthood. For example, when referring to rats, adolescent rats are meant to include rats from about 4 - 10 weeks old; when referring to humans, adolescent is referred to as the period between about 10 - 20 years of age (as defined by the WHO).

In the context of the present invention, the term `adult' is meant to be the phase of adulthood during a mammals life. For example, when referring to rats, adult rats are meant to include rats from about 10 to 40 weeks old; when referring to humans, adults are used in the context of a person being about 18 years or older.

In the context of the present invention, beta cell precursor cells are meant to include adult progenitor cells as well as mammalian cells capable to trans-differentiate into endocrine cells.

During the screening method of the present invention, different glucose concentrations will/can be employed, partly dependent on the type of beta cells (or precursors thereof) being used, and in a further aspect to assess the effect of test compounds under different conditions. Exposing the cells to a sub-optimal glucose concentration could for example be interesting to asses the presence or absence of a supplementary effect of a compound to be tested. Thus in a further aspect the glucose concentration used in the methods of the present invention can vary from about 1 - about 50 mM glucose, in particular about 10 - about 20 mM, more in particular about 2.5 mM, about 5 mM, about 10 mM or about 20 mM.

Suitable assay conditions may be dependent on the type and age of the cells used. For example when using adolescent rat cells (rats being 8-20 weeks of age), glucose concentrations may for example be selected from about 2.5 mM, about 5 mM, about 10 mM or about 20 mM. Similar assay conditions are applicable to human cells (humans being for example 4 - 70 years of age).

A further parameter useful in exposing the beta cell to selected conditions, has to do with the screening term. In principle beta cell activity can be monitored over any suitable time-interval. The step of comparing beta cell activity over time is performed after about 1-15 days of culture, in particular after about 1, 3, 6, 7 or 9-15 days,. Suitable time-intervals depend on the parameter to be detected. For example beta cell phenotype can be determined at any time point during incubation, such as for example on day 1, 3, 6, 7, 9 and 15.

As shown in more detail hereinafter, using the aforementioned screening method the applicant has been able to screen compound libraries, and identified compounds that enhance beta cell number and/or phenotype. Evidently, compounds thus identified are subsequently useful as positive controls in the aforementioned screening methods. It is accordingly an object of the present invention to provide the use of a compound identified in any one of the aforementioned screening methods, as a positive control in a method for identifying compounds that influence beta cell number and/or phenotype; more in particular for identifying compounds that influence state of differentiation; state of structural integrity; state of proliferative activity; viability; or state of functional activity such as insulin biosynthesis, insulin secretion, or glucose responsiveness.

In a particular embodiment, the method according to the present invention, may also be used to study inhibition of a compound capable of inducing proliferation.

Using the beta cell screening method of the present invention, it has also been established that the enhancement of beta cell number and/or phenotype, more in particular beta cell numbers and beta cell proliferation follows from triggering the transactivation pathway of the glucocorticoid receptor in said cells. In other words, compounds capable of binding with the glucocorticoid receptor thereby activating the glucocorticoid receptor transactivation pathway will enhance beta cell number and/or phenotype and are accordingly useful in the aforementioned application.

Hence, in a further embodiment the present invention provides the use of the glucocorticoid receptor in identifying compounds that influence beta cell number and/or phenotype, i.e. in identifying compounds capable to enhance beta cell numbers and to induce beta cell proliferation, more in particular compounds that simulate beta cell differentiation, proliferation. In one aspect said use consists of a method to identify such compounds, by determining the ability of said compound to induce the glucocorticoid receptor dependent-transactivation pathway in said beta cells, wherein a compound that is capable of inducing the glucocorticoid receptor-dependent transactivation pathway, is identified as a compound that stimulates beta cell differentiation, proliferation and/or expansion. Said method is hereinafter referred to as the 'transactivation assay'.

Induction of the glucocorticoid receptor-dependent transactivation pathway can be measured using different methods/techniques. For example, the compound to be tested can be contacted with the glucocorticoid receptor followed by determining the capability of said compound to bind with said receptor. Said assessment may for example also be based on a (dual) luciferase assay for determining interaction of a compound with the ligand binding site of the receptor.

However, further interesting compounds may also indirectly influence the glucocorticoid receptor-dependent transactivation pathway, i.e. without binding to the glucocorticoid receptor. Hence, qPCR based detection methods may be used to determine the effect of a compound on the expression of transrepression and/or transactivation target genes, either after binding to the glucocorticoid receptor or by indirect induction of the transactivation pathway in suitable cell lines.

In the context of the present invention, said compounds may either by steroidal or nonsteroidal compounds.

Hence, in a particular aspect, the present invention provides a method as defined above, wherein testing the ability of said compound to induce the glucocorticoid receptor dependent-transactivation pathway in said beta cells comprises the steps of:
- culturing beta cells or precursors thereof in the absence (control sample), or presence (test sample) of a test compound,
- measuring the expression of one or more transactivation markers in said beta cells or precursors thereof in both samples;
wherein a compound that increases expression of said one or more transactivation markers in said test sample in comparison with said control sample, is identified as a compound that stimulates beta cell differentiation, proliferation and/or expansion.

In the context of the present invention, the one or more transactivation markers may be selected from the non-limiting list comprising FKBP5, MKP1, IL-1Ra, Fgb and Fgg. In the context of the present application, the one or more transactivation markers are preferably selected from the non-limiting list comprising; FKBP5, MKP1 and IL-1Ra.

In a further aspect, the method according to this invention, may further comprise the step of measuring the expression of one or more transrepression markers in said beta cells;
wherein a compound that increases expression of one or more transactivation markers, and does not decrease the expression of one or more transrepression markers in said test sample in comparison with said control sample; is identified as a compound that stimulates beta cell differentiation, proliferation and/or expansion

In the context of the present invention, the one or more transrepression markers may be selected from the non-limiting list comprising ICAM1, CXCL11, Nfkbie, Myd88, Birc3. In the context of the present application, the one or more transrepression markers are preferably selected from the non-limiting list comprising; ICAM1 and CXCL11

Preferably, the compounds according to the present invention exhibit at least one of the following effects on the glucocorticoid receptor pathways:
- transactivation and transrepression
- transactivation and no transrepression

Most preferably, the compounds according to the present invention show transactivation, while they have no effect on transrepression of the glucocorticoid receptor-dependent pathways.

In one embodiment the aforementioned 'transactivation' assay can be combined with the screening assay using the culturing of primary mammalian beta cells or precursors thereof in (serum-free) medium comprising glucose. Compounds initially identified as being capable of inducing the glucocorticoid receptor-dependent transactivation pathway can be tested subsequently for their ability to influence beta cell number and/or phenotype in the cellular assays as herein provided. Addition of a glucocorticoid receptor agonist in the screening assays as provided herein will result in stimulation of beta cell proliferation. Such proliferation may for example be determined by incorporation of a thymidine analogues measured at different time points after addition of the agonist; e.g. incorporation of EdU or BrdU measured between 3-6 days after addition of the analogue. In addition, the difference in increase in absolute beta cell numbers over a give time frame (e.g. 15 days) between the basal control condition (- agonist) and the test condition (+ agonist) may be determined.

Hence, in a specific aspect, the present invention provides a method for identifying a compound that stimulates beta cell proliferation over time, said method comprising the steps of:
- providing a compound that is capable of inducing the glucocorticoid receptor dependent-transactivation pathway in beta cells or precursors thereof
- culturing beta cells or precursors thereof in the presence of said compound and a thymidine-analog
- measuring the degree of incorporation of said thymidine-analog in said beta cells over time;
wherein a compound that increases thymidine incorporation in said beta cells over time, is identified as a compound that influences beta cell proliferation

Said method is hereinafter referred to as the 'proliferation assay'.

In addition to measuring the effect of a compound on proliferation of beta cells, they may also be further tested for their ability to stimulate beta cell differentiation and/or expansion.

Hence, in a further aspect, the present invention provides a method for identifying a compound that stimulates beta cell differentiation over time, said method comprising the steps of:
- providing a compound that is capable of inducing the glucocorticoid receptor dependent-transactivation pathway in beta cells or precursors thereof
- culturing beta cells or precursors thereof in the presence of said compound
- measuring over time in said beta cells the expression of one or more differentiation markers, selected from MafB, DLK1, NPY, NNAT and TRH; and one or more maturation markers selected from MafA and PDX1 ;
wherein a compound that increases expression of at least one of said differentiation markers and does not decrease the expression of said one or more maturation markers ,over time, is identified as a compound that stimulates beta cell differentiation

Said method is hereinafter referred to as the 'differentiation assay'.

The present invention also provides a method for identifying a compound that stimulates beta cell expansion over time, said method comprising the steps of:
- providing a compound that is capable of inducing the glucocorticoid receptor dependent-transactivation pathway in beta cells or precursors thereof
- culturing beta cells or precursors thereof in the presence of said compound
- measuring the absolute number of living beta cells over time using high content imaging, wherein a compound that increases the absolute number of living beta cells over time is identified as a compound that stimulates beta cell expansion

Said method is hereinafter referred to as the 'expansion assay'.

Preferably, said compounds that are capable of inducing the glucocorticoid receptor dependent-transactivation pathway in beta cells or precursors thereof, are identified using the 'transactivation assay' according to this invention.

The present application thus provides a method for identifying a compound suitable for the treatment of pathologies characterized by a change in beta cell differentiation, proliferation and/or expansion, such as for example diabetes mellitus; said method comprising the steps of:
- performing the 'transactivation assay' as provided herein, and
- performing the 'proliferation assay' as provided herein
wherein a compound that is capable of inducing the glucocorticoid receptor dependent-transactivation pathway in beta cells or precursors thereof as determined using the 'transactivation assay', and is capable of stimulating beta cell proliferation as determined using the 'proliferation assay'; is identified as a compound that is suitable for the treatment of pathologies characterized by a change in beta cell differentiation, proliferation and/or expansion, such as for example diabetes mellitus.

The present application provides a method for identifying a compound suitable for the treatment of pathologies characterized by a change in beta cell differentiation, proliferation and/or expansion, such as for example diabetes mellitus; said method comprising the steps of
- performing the 'transactivation assay' as provided herein,
- performing the 'proliferation assay' as provided herein, and
- performing the 'expansion assay' as provided herein
wherein a compound that is capable of inducing the glucocorticoid receptor dependent-transactivation pathway in beta cells or precursors thereof as determined using the 'transactivation assay', is capable of stimulating beta cell proliferation as determined using the 'proliferation assay', and is capable of stimulating beta cell expansion using the 'expansion assay'; is identified as a compound that is suitable for the treatment of pathologies characterized by a change in beta cell differentiation, proliferation and/or expansion, such as for example diabetes mellitus.

The present application provides a method for identifying a compound suitable for the treatment of pathologies characterized by a change in beta cell differentiation, proliferation and/or expansion, such as for example diabetes mellitus; said method comprising the steps of
- performing the 'transactivation assay' as provided herein,
- performing the 'differentiation assay' as provided herein, and
- performing the 'proliferation assay' as provided herein,
wherein a compound that is capable of inducing the glucocorticoid receptor dependent-transactivation pathway in beta cells or precursors thereof as determined using the 'transactivation assay', is capable of stimulating beta cell differentiation as determined using the 'differentiation assay', and is capable of stimulating beta cell proliferation as determined using the 'proliferation assay', is identified as a compound that is suitable for the treatment of pathologies characterized by a change in beta cell differentiation, proliferation and/or expansion, such as for example diabetes mellitus.

In the most preferred embodiment, the present invention provides a method for identifying a compound suitable for the treatment of diabetes mellitus; said method comprising the steps of
- performing the 'transactivation assay' as provided herein,
- performing the 'differentiation assay' as provided herein,
- performing the 'proliferation assay' as provided herein, and
- performing the 'expansion assay' as provided herein
wherein a compound that is capable of inducing the glucocorticoid receptor dependent-transactivation pathway in beta cells or precursors thereof as determined using the 'transactivation assay', is capable of stimulating beta cell differentiation as determined using the 'differentiation assay', is capable of stimulating beta cell proliferation as determined using the 'proliferation assay', and is capable of stimulating beta cell expansion using the 'expansion assay'; is identified as a compound that is suitable for the treatment of pathologies characterized by a change in beta cell differentiation, proliferation and/or expansion, such as for example diabetes mellitus.

The present application further provides a method for producing a pharmaceutical composition comprising combining a compound that is capable of inducing the glucocorticoid dependent-transactivation pathway, with a pharmaceutically acceptable carrier.

The present application also provides a method for producing a pharmaceutical composition for the treatment of diabetes mellitus, comprising combining a compound that is capable of inducing the glucocorticoid dependent-transactivation pathway, with a pharmaceutically acceptable carrier.

In particular, in said methods, the compound that is capable of inducing the glucocorticoid dependent-transactivation pathway, is identified using the 'transactivation assay' as defined herein.

Having identified the glucocorticoid receptor transactivation pathway as one of the possible pathways for influencing beta cell number and/or phenotype, a further screen could be established to allow and identify compounds capable of influencing beta cell number and/or phenotype through a different pathway. In said instance, the compounds may be tested in the presence of an antagonist of the glucocorticoid receptor transactivation pathway, such as for example mifepristone.

Further to their application as positive control in each of the aforementioned screening methods, the compounds identified in the beta cell screening assays could also be used in;
- their application of influencing beta cell number and/or phenotype *in vitro* or *in vivo;* wherein the beta cell phenotype is in particular based on one or more parameters selected from state of differentiation; state of structural integrity; state of proliferative activity; viability; or state of functional activity such as insulin biosynthesis, insulin secretion, or glucose responsiveness.
- the treatment of pathologies characterized by a change in beta cell numbers and/or phenotype such as for example diabetes mellitus
- their application in increasing beta cell number *in vitro* for transplantation purposes.

It is accordingly an object of the present application to provide the use of the compounds identified using the screening methods of the present invention in the aforementioned applications, including said compounds for use in the treatment of pathologies characterized by a change in beta cell numbers and/or phenotype such as for example diabetes mellitus. Exemplary compounds identified as capable of influencing, more in particular enhancing beta cell number and/or phenotype, are glucocorticoids.

A detailed description of the culture method according to this invention, can be found in the examples that follow herein after, and is shortly summarized as follows:

### Purification and culture of (rat) beta cells

- Beta cells are purified from islet-cell enriched suspensions, this may for example be achieved by FACS sorting as described in (Pipeleers et al., 1985a; Van de Winkel et al., 1982). In the context of the present invention neonatal rats are considered about 2-3 days old, adolescent rats are about 4-10 weeks old and adults are about 10-40 weeks of age.
- Purified cells are seeded (day 0) for example in 804G matrix-coated plates, laminine-coated plates or collagen type IV coated plates; and subsequently cultured for up to 15 days in serum-free Ham's-F10 medium containing 5, 10 or 20mmol/l glucose. The medium is preferably further supplemented with 2mmol/l L-glutamine, 50µM 3-isobutyl-1-methylxanthine, 0.5% Albumax-I, penicillin (100U/ml) and streptomycin (0.1 mg/ml). Albumax may be replaced by a suitable bovine serum albumin analog.
- Culture medium and cells may be harvested after different time-points and the suitable parameters can subsequently be determined.

Hence, in a preferred embodiment, the beta cells or precursors thereof are cultured in Ham F10 medium supplemented with about 1 - about 50 mM glucose, about 0 - about 50 µM IBMX (isobutyl-1-methylxanthine), about 0.1% - about 4% albumax I, about 0.1 mg/ml streptomycin, and about 0.075 mg/ml penicillin. Furthermore, the beta cells or precursors thereof are preferably cultured on extracellular matrices, such as for example 804G matrices.

In a specific embodiment, the beta cells or precursors thereof according to this invention, are isolated from a perinatal mammal, adolescent mammal, or adult mammal; more in particular from human, porcine, rat, or mouse.

### Measurement of number, survival and proliferation activity in cultured beta cells

- On day 1, 9 and 15 of culture, total numbers of living beta cells per well can be determined such as for example by semi-automatic cell count, after Hoechst 33342 (Ho) and propidium iodide (PI) staining.
- Numbers of living beta cells may be obtained by subtracting the PI-positive cell numbers from the Ho-positive cell numbers, and correcting for the percent insulin-negative cells, as determined following insulin-immunofluorescence staining. They can then be expressed as percent of the numbers on day 1.
- Each condition can further be evaluated for increases in the percentage of proliferating beta cells for example by incorporation of the thymidine-analogue 5-ethynyl-2'-deoxyuridine, EdU; (100µmol/l).
- To detect beta cells that underwent two successive cycles, cells can sequentially be exposed to two different thymidine-analogs, such as for example first EdU from day 3 to 6, and then BrdU (100µmol/l) from day 7 to 10, with a 24-hour label-free period in between. EdU-positive (EdU⁺) nuclei can for example be detected with the Click-iT Assay kit (Invitrogen), while BrdU-positive (BrdU⁺) nuclei can be identified with anti-BrdU antibody.

Hence, in a particular embodiment, the measuring over time is performed after about 1-15 days of culture, in particular after about 1, about 3, about 6, about 9, about 12 or about 15 days of culture.

### Data acquisition and analysis

Absolute number of cells can be determined for example by whole-well imaging using the BD-PathwayBioimager855 (BD-Biosciences). IPLab and AttoVision software packages (BD-Biosciences) can be used for image background subtraction, segmentation and quantification.

### Statistics

Data can be presented as means ± standard error of the mean (SEM) of n independent experiments. Each condition preferably consists of triplicate samples and is preferably tested at least three times. Statistical differences between means can be assessed with two-tailed unpaired Student's t test or ANOVA with Tukey's test for multiple comparisons using GraphPad Prism (GraphPad Software, San Diego, USA).

### EXAMPLES

The following examples illustrate the invention. Other embodiments will occur to the person skilled in the art in light of these examples.

### Example 1: Glucose Regulates Rat Beta Cell Number through Age-dependent Effects on Beta Cell Survival and Proliferation

### Materials

Culture medium and supplements were purchased from Invitrogen (Life Technologies Ltd, Paisley, UK), glucokinase activator, RO-28-1675, from Axon Medchem (Groningen, The Netherlands). Anti-insulin antibody was prepared in our laboratory, anti-bromo-deoxyuridine (BrdU) antibody purchased from DakoCytomation (Glostrup, Denmark) and Hoechst 33342 and propidium iodide from Sigma. The 804G cell line and the method for preparation of its matrix have been described before (Bosco et al., 2000). The cells were kindly provided by Dr T. Otonkoski, University of Helsinki, Finland. Isolated beta cells were seeded in 804G matrix-coated 384-well black plates (Greiner Bio-One, Frickenhausen, Germany).

### Purification and culture of rat beta cells

Beta cells were FACS-purified from islet-cell enriched suspensions (Pipeleers et al., 1985) prepared from neonatal (2-3 days-old) and adolescent or adult (8 or 40-week-old, male) Wistar rats (Janvier Bioservices, France); their purity was, respectively 70-75% and over 90%. Sorted cells were counted in a hemocytometer, seeded (day 0) and cultured for up to 15 days in serum-free Ham's-F10 medium at 5, 10 or 20mmol/l glucose, supplemented with 2mmol/l L-glutamine, 50µM 3-isobutyl-1-methylxanthine, 0.5% Albumax-I, penicillin (100U/ml) and streptomycin (0.1 mg/ml). The 10mmol/l glucose condition is known to maintain beta cell survival over 9-days (Ling et al., 1994; Ling and Pipeleers, 1994) . Medium and cells were retrieved after selected culture periods and assayed for insulin content; data were expressed as a function of the number of living beta cells that was determined in parallel wells.

### Measurement of number, survival and proliferation activity in cultured beta cells

On day 1, 9 and 15 of culture, total numbers of living beta cells per well were determined by semi-automatic cell count, after Hoechst 33342 (Ho) and propidium iodide (PI) staining. Numbers of living beta cells were obtained by subtracting the Pi-positive cell numbers from the Ho-positive cell numbers, and correcting for the percent insulin-negative cells, as determined following insulin-immunofluorescence staining. They were expressed as percent of the numbers on day 1. Each condition was also evaluated for increases in the percentage of proliferating beta cells (incorporating the thymidine-analogue 5-ethynyl-2'-deoxyuridine, EdU; 100µmol/l). To detect beta cells that underwent two successive cycles, cells were sequentially exposed to two different thymidine-analogs, first EdU from day 3 to 6, and then BrdU (100µmol/l) from day 7 to 10, with a 24-hour label-free period in between. EdU-positive (EdU⁺) nuclei were detected with the Click-iT Assay kit (Invitrogen), while BrdU-positive (BrdU⁺) nuclei were identified with anti-BrdU antibody.

### Data acquisition and analysis

Absolute number of cells was determined by whole-well imaging using the BD-PathwayBioimager855 (BD-Biosciences). IPLab and AttoVision software packages (BD-Biosciences) were used for image background subtraction, segmentation and quantification. The method was validated for 96- and 384-well plates in which living and dead cells, insulin-positive cells and nuclei with thymidine-analogues were counted.

### Statistics

Data are presented as means ± standard error of the mean (SEM) of n independent experiments. Each condition consisted of triplicate samples and was tested at least three times. Statistical differences between means were assessed with two-tailed unpaired Student's t-test or ANOVA with Tukey's test for multiple comparisons using GraphPad Prism (GraphPad Software, San Diego, USA). Differences were considered significant at *p*<0.05.

### Results

### Proliferation assay by imaging-based cell counts

In order to validate imaging-based cell counts, a known number of beta cells, as determined in the hemocytometer, was seeded in 384-well plates and semi-automatically counted after one day of culture. Whole-well images were acquired and quantified by an operator-independent system in Figure 1. A linear correlation was found between seeded and counted cell numbers (adjusted r²=0.99) over a large range (100 to 10,000 cells per well; Fig. 1A). Above 10,000 cells per well, precision decreased since cell aggregation limited segmentation of individual cells. Subsequent experiments were therefore conducted with 2000 cells seeded per well (18000 cells/cm²). The method distinguishes the numbers of living and of dead cells using propidium iodide as marker for dead cells. When cells were cultured for 2-days with cycloheximide, a known inducer of apoptosis in beta cells, an inverse correlation was found between the numbers of living and dead cells (Hoorens et al., 1996), while the total numbers remained constant (Fig. 1B). These results indicate that the assay can detect conditions of beta cell death and assess their influence on the number of living beta cells in culture.

### Glucose induces dose-dependent increase in number of adult rat beta cells during culture

Two weeks culture at 5mmol/l glucose decreased the number of living beta cells by 21 percent (Table 1). This effect can be attributed to apoptosis which occurs at this glucose concentration but not at 10mmo/l (Hoorens et al., 1996). Culture at 10 and 20mmol/l glucose increased beta cell numbers by, respectively, 16 and 31 percent (Table 1). This increase was time-dependent; after 9 days, it was not yet observed at 10mmoi/i, while in part present at 20mmol/l glucose (21 percent increase). It not only reflects glucose-induced suppression of apoptosis but also induction of replication. There was no increase in the number of insulin-negative cells (> 88% insulin-positive cells on day 15); an increase in total cell number is thus indicative for beta cell replication.

**Table 1: Effect of glucose on the number of living beta cells.**

| **Culture Condition** | **Number of Living Beta Cells (% of day 1)** | | |
|---|---|---|---|
| | Day 1 | Day 9 | Day 15 |
| 5mmol/l glucose | 1744 ± 15 (100 ± 3) | 1503 ± 100 (86 ± 6) | 1376 ± 93**,≠ (79 ± 5) |
| 10mmol/l glucose | 1734 ± 15 (100 ± 4) | 1897 ± 56 (109 ± 3) | 2027 ± 56** (116 ± 3) |
| 20mmol/l glucose | 1752 ± 15 (100 ± 3) | 2126 ± 123** (121 ±7) | 2308 ± 72***,# (131 ± 4) |

| | | | |
|---|---|---|---|
| Adult rat (8-week-old) beta cells were cultured in serum-free medium at the indicated concentrations of glucose. The numbers of viable beta cells are expressed as absolute cell number per well and as percent of the numbers on day 1. Data represent means ± SEM; compared with day 1 values: *, p<0.05; **, p<0.01; ***, p<0.001; compared with 10mmol/l glucose condition on day 15: ≠, p<0.001; #, p<0.01; n=8. | | | |

### Glucose-induced recruitment of adult rat beta cells into DNA-synthetic activity reveals heterogeneity in cell responsiveness

To evaluate the kinetics of glucose-induced proliferation, beta cells seeded at 10 or 20mmol/l were cultured with the thymidine-analogue EdU for selected time slots (48h at start between day 1 and 3, and 72h time slots thereafter, Fig. 2). At 10mmol/l glucose, percent EdU⁺ beta cells was low on day 3 (1.1±0.1%), time-dependently increased up to 8.5±0.7% on day 12 and then decreased (to 2.7±0.1% on day 15). At 20mmol/l, the percentage was only slightly higher on day 3 (2.0±0.2%) but three-fold on day 6 (11.3±0.4%) reaching its maximum and maintaining it during the subsequent three days; it then decreased to similar levels as at 10mmol/l (Fig. 2). The sum of the percentages counted during the subsequent intervals (24% at 10 and 34% at 20mmol/l glucose) could overestimate the total percent recruited cells as it might contain two EdU-positive daughter cells for cells that have been activated during the prior period. On the other hand, some daughter cells may have re-entered a new cycle, and should thus be distinguished from activated quiescent cells. The data on EdU incorporation are nevertheless in line with the observed increases in cell number (Table 1). Moreover, they provide evidence that glucose only recruited a fraction of beta cells into DNA-synthesis. This recruitment required several days of exposure to stimulating glucose concentrations with a more rapid effect at 20mmol/l.

To examine whether newly formed beta cells were the progeny of a small pool of repetitively replicating cells or a result of time-dependent recruitment of new beta cells into the cell cycle, we performed sequential labeling with two thymidine-analogues (Teta et al., 2007). It has been previously shown that prior incorporation of a thymidine-analog in beta cells does not diminish their probability for cell cycle re-entry (Salpeter et al., 2010; Teta et al., 2007), which we confirmed in INS1832/13 cells (data not shown). Purified rat beta cells were first incubated with EdU from day 3 to 6, and then with BrdU from day 7 to 10. The percent EdU⁺ cells that were BrdU⁺ on day 10 represents the fraction of cells that re-entered the cell cycle during the second period. This fraction was only 0.5±0.1% of all cells at 10mmol/l and 1.1±0.2% at 20mmol/l glucose, far less than the percentages that had entered the cell cycle during the first three day period (respectively, 5.8±0.4% and 8.1±1.2%; Table 2). Cell cycle re-entry within 3 days is thus a rare event in the present conditions. On the other hand, the corresponding percentages of only BrdU⁺ cells (8.8±0.7 and 17.7±2.3%) indicate that glucose continues to recruit beta cells into proliferation during this second period, mostly from the subpopulation that was not activated during the first period and more so at 20mmol/l glucose. It is thus concluded that the time-dependent increase in the number of beta cells (Table 1) does not result from a small pool of repetitively replicating cells but mainly from recruitment of progressively more quiescent cells into the cell cycle.

In view of the higher percent double-labeled cells at 20mmol/l glucose, we examined whether this reflects a shortened cell cycle re-entry period.. A mathematical model was used to estimate the probability that a replicated beta cell re-enters the cell cycle. To this end, the fraction of EdU⁺ beta cells at day 10 was multiplied by the corresponding fraction of BrdU⁺ cells. If beta cell replication occurs stochastically, this predicted value should equal the counted percent EdU⁺BrdU⁺ beta cells; it will be lower if the cell cycle re-entry period is shortened, as in a continuously replicating subpopulation. Our data suggest that the counted fractions of EdU⁺BrdU⁺ cells were significantly lower than the predicted values, both at 10 and 20mmol/l glucose (Fig. 3), indicating a prolonged refractory period in both conditions. The higher fraction of double-labeled beta cells at 20mmol/l glucose is thus attributable to a higher number of cells that has been recruited into the S-phase, as is also reflected by the higher percent of only BrdU⁺ cells in this condition (Table 2).

**Table 2: Effect of glucose on recruitment of beta cells into DNA-synthesis and on cell cycle re-entry of previously recruited cells.**

| **Culture Condition** | **Day 6** | | **Day 10** | |
|---|---|---|---|---|
| | % EdU⁺ | % EdU⁺BrdU⁻ | % BrdU⁺EdU⁻ | % EdU⁺BrdU⁺ |
| Adult beta cells | | | | |
| 10mmol/l glucose | 5.8 ± 0.4 | 8.0 ± 0.4 | 8.3 ± 0.7 | 0.5 ± 0.1 |
| 20mmol/l glucose | 8.1 ± 1.2 | 9.5 ± 1.6 | 16.6 ± 2.1** | 1.1 ± 0.2* |
| Neonatal beta cells | | | | |
| 10mmol/l glucose | 28.7 ± 2.2*** | 27.1 ± 3.7*** | 23.1 ± 5.2** | 11.3 ± 1.6** |

| | | | | |
|---|---|---|---|---|
| Adult (8-week-old; n=5) and neonatal (2-3 days-old; n=3) beta cells were cultured at the indicated glucose concentrations for 10 days with sequential labeling with EdU (day 3 to day 6) and BrdU (day 7 to day 10). The percentages of EdU⁺ and/or BrdU⁺ beta cells are shown as means ± SEM; compared with adult 10mmol/l glucose: *, p<0.05; **, p<0.01; ***, p<0.001. | | | | |

### Effect of age on glucose-induced increases in beta cell number

The effects of glucose on beta cells from adolescent rats (8-weeks) were compared with those from neonatal (2-3 days old) and 40-week-old rats (Table 3). The number of living beta cells from 40-week-old rats did not vary significantly over the two-week culture period; in contrast to the 8-week-old rats, there was no decrease at 5mmol/l glucose and no increase at 10 and 20mmol/l. On the other hand, the number of neonatal beta cells doubled at 5 and 10mmol/l glucose and decreased at 20mmol/l (Table 3). At the highest glucose concentration, a marked fraction of the neonatal beta cells died, which was not, or only marginally the case for beta cells from the older age groups (Table 4). In presence of the GKA (3 µm), they underwent a marked cytotoxicity (>50% dead cells at day 15) at 5 mmol/l glucose, which was not observed in beta cells isolated from 8- and 40-week old rats (data not shown).

**Table 3: Age-dependent proliferation of rat beta cells in response to various doses of glucose.**

| **Culture Condition** | **Number of Living Beta Cells on Day 15 (% of day 1)** | | |
|---|---|---|---|
| | **neonatal** | **8-week-old** | **40-week-old** |
| 5mmol/l glucose | 193±12* | 74±5** | 96±11 |
| 10mmol/l glucose | 198±12*,≠ | 118±5** | 110±5 |
| 20mmol/l glucose | 81±14*,# | 127±5*** | 117±10 |

| | | | |
|---|---|---|---|
| Beta cells isolated from the different age groups were cultured at the indicated concentrations of glucose and the numbers of viable beta cells were determined after 15 days in culture and expressed as percent of the numbers on day 1. Data represent means ± SEM; *, p<0.05; **, p<0.01; ***, p<0.001 versus corresponding day 1 values; ≠, p<0.05; #, p<0.01 versus 8-week-old at 10mmol/l glucose; n=3-8. | | | |

**Table 4: Effect of age on susceptibility of beta cells to glucose toxicity.**

| | | % **Living Beta Cells on Day 15** | | |
|---|---|---|---|---|
| **Culture Condition** | | **neonatal** | **8-week-old** | **40-week-old** |
| | 5mmol/l glucose | 88±2 | 83±2 | 92±2 |
| | 10mmol/l glucose | 84±1** | 96±0 | 94±4 |
| | 20mmol/l glucose | 69±4*** | 92±1 | 86±1** |

| | | | | |
|---|---|---|---|---|
| Beta cells purified from neonatal (n=3) 8 week (n=8) and 40 week (n=3) old rats were cultured for 15 days at the indicated glucose concentrations. The percent living beta cells was determined by the propidium iodide assay and expressed as means ± SEM; **, p<0.01; ***, p<0.001 versus 8-week old rats at corresponding glucose concentration. | | | | |

The ability of neonatal beta cells to double their number during a two-week culture involved, as in adult beta cells, time-dependent recruitment of beta cells into DNA-synthesis. This is demonstrated by EdU-BrdU sequential labeling of the cells, cultured at 10mmol/l glucose (Table 2). Compared to the 8-week-old rats, the percentages of activated neonatal cells were significantly higher during both labeling phases (28.7±2% EdU⁺ between day 3 and 6, and 23.1±5.2% BrdU+EdU- cells between day 7 and 10. Thus, 52% of neonatal beta cells on day 10 were newly formed, versus only 14% for 8-week-old rats (Table 2). In addition, a markedly larger fraction of cells activated during the first phase re-entered the cell cycle during the second period (24% of EdU⁺ neonatal cells on day 10 were also BrdU⁺ versus only 5% in adolescent beta cells). When expressed as percentage of total beta cell number on day 10, 11% of neonatal beta cells had participated in at least two replicative cycles, 20-fold more than in adolescent beta cells (Table 2). However, when comparing the predicted and counted values for double-positive cells, no difference was found suggesting that neonatal beta cells that had been recruited into proliferation were not preferentially activated towards re-entry nor have they been primed for a prolonged refractory period (Fig. 3). The higher fraction of neonatal beta cells that re-enters the cell cycle is thus a consequence of the higher percent cells that enter proliferative activity. This was the case for 50% of the neonatal beta cells, which is in line with the doubling in cell number. Since a similar increase in beta cell number was seen at basal glucose concentration (5mmol/l), there is no evidence that this replication is the result of a dose-dependent glucose-regulated recruitment of beta cells into proliferation, as was the case for the adolescent beta cell population. In fact, beta cell number decreased during culture at 20mmol/l glucose, the result of higher percentages of dead beta cells (Table 3) which are indicative for the higher susceptibility of neonatal cells to glucotoxicity.

### Discussion

Glucose is since long considered to exert influences on the beta cell mass. The nutrient has been reported to positively or negatively influence the survival (Heit et al., 2006; Hoorens et al., 1996; Ling et al., 1994)and/or the proliferation (Heit et al., 2006; Salpeter et al., 2010; Salpeter et al., 2011)of beta cells. Variability in its effects might be attributable to differences in concentration and duration, and/or in environmental conditions but also to differences in readout parameters, often without data on beta cell numbers. The present in vitro study shows that glucose can increase the number of beta cells that have been purified from adolescent rats. This effect involves a time- and dose-dependent activation of beta cells into DNA-synthesis, recruiting, over a two-week culture period up to 34 percent of the cells and leading to a 30 percent increase in numbers of living cells. It was not observed at basal 5mmol/l glucose, a condition in which beta cell numbers decrease as result of inadequate protein and mitochondrial activities (Hoorens et al., 1996). The observed increases in living beta cell numbers at 10 and 20mmol/l are thus the result of two effects, a glucose-induced preservation of beta cell survival and a glucose-induced proliferation. Using these specific conditions, the expanded beta cell population did not exhibit obvious differences in its phenotype: mRNA expression of the beta cell characteristic proteins Ins 1, Ins2, Nkx6.1, glut 2, glucokinase was similar than in the population before replication, the average cellular insulin content was not decreased, neither the insulin producing capacity as judged by the amount of insulin released over the preceding 72 hours and expressed per million beta cells. The data do not support the concept that "switch factors" regulate a shift between insulin production and proliferation (Liu et al, 2009), but do not exclude it in view of the fact that they were collected for the entire beta cell population whereas only 20 to 30 percent of the cells appeared recruited into a replicative activity.

Only a fraction of the beta cells from adolescent rats underwent replication during the two-week culture. Its recruitment was not synchronous but presented intercellular differences in time to activation. This dissimilarity in glucose-induced proliferation is another illustration of the functional heterogeneity in the beta cell population (Pipeleers et al., 1994). It should nevertheless be distinguished from the previously reported intercellular differences in glucose-induced insulin synthesis. While the latter can, to a large extent, be overcome by several days of culture at high glucose (Pipeleers et al., 1994), this was not the case for activation of proliferation, with a majority of cells remaining unresponsive during the two-week study period. We examined whether responsive cells belonged to a subpopulation which, when activated into proliferation, would proceed into successive cell cycles. Using sequential EdU-BrdU labeling, this appeared not the case: beta cells entering a second cycle were rare. Glucose-induced replication thus resulted from recruitment of quiescent beta cells into the cell cycle instead of activating a limited pool into repetitive replication. In fact, subsequent re-entry into a new cycle was restricted by a "refractory" period, making a post-mitotic beta cell less likely to replicate than a resting beta cell. While our data do not support the existence of a limited replicative pool of beta cells, they neither demonstrate that all beta cells exhibit the same potential to proliferate (Salpeter et al., 2010; Teta et al., 2007). The latter is however not excluded since it may depend on synergistic stimuli as was also noticed for glucose-induced insulin release (Pipeleers et al., 1985b) .

Beta cells isolated from old rats did not exhibit a glucose-induced increase in beta cell number at 10 or 20mmol/l glucose. They were also not susceptible to cell death during culture at 5mmol/l glucose; it needs to be investigated whether this reflects enhanced beta-cell sensitivity to glucose as suggested in a study on insulin release in aging rats (Ruhe et al., 1992). Consequently, their cell number remained constant over the two-week culture period. Our data are consistent with prior work showing an age-dependent decline in the replicative capacity of beta cells in rodents and humans. On the other hand, neonatal beta cells replicated in absence of elevated glucose concentrations, and to a much higher extent than adolescent beta cells during glucose activation; their number doubled within two weeks. The majority of neonatal cells appeared primed to proliferate at basal 5mmol/l glucose without further increase at 10mmol/l. Proliferating neonatal beta cells did not exhibit a refractory period, which is at variance with adolescent cells; they can thus re-enter a new cell cycle as rapidly as quiescent cells. Our data are not indicative for the presence of a specialized pool of continuously replicating beta cells, but clearly indicate a major shift in the conditions under which beta cells proliferate between birth and adolescent age, i.e. from a population where most cells replicate at basal glucose to one where a minority of cells replicates at elevated glucose concentrations. A delay or acceleration in this transition might have important implications on the size of the beta cell population later in life. These observations also underline the need to further compare phenotypes and metabolic pathways in beta cells in this time window.

Neonatal beta cells have been reported to lack the glucose responsiveness as seen in adult beta cells. They appear on the other hand highly susceptible to glucotoxicity, which explains the failure to expand their population at 20mmol/l glucose.. When cultured at this concentration, a progressive increase in the number of dead cells was noticed, leading to a final number of living beta cells that was 20 percent lower than at start. This observation might have pathophysiologic relevance as it raises the possibility that beta cells early in life are particular susceptible to metabolic alterations, with consequences on their survival and on the size of the developing beta cell mass.

### Example 2: Glucocorticoids increase replication in adult rat beta cells

In example 1, we described the development and potential application of a high-content, image-based drug screening assay for the identification of compounds that can induce a proliferation activity in beta cells. The approach represents an alternative to gene manipulation and overexpression of potentially carcinogenic cell cycle-regulating genes. In the current example, we used this new assay to screen a commercially available library of compounds for agents that can induce proliferation specifically in primary beta cells, leading to a demonstrable increase in the absolute number of cells. Freshly isolated beta cells retain most of the in vivo behaviors of mature cells and FACS-purification eliminates other cell types that can potentially influence beta cell properties and their response to treatment. The screening platform where multiple parameters (cell viability, induction of proliferation and changes in cell numbers) are measured in a single experiment enabled us to eliminate potential hits from compounds that were also cytotoxic and would therefore be unsuitable in the long run.

### MATERIALS AND METHODS

**Materials:** Cell culture medium and all medium supplements were purchased from Invitrogen (Life Technologies Ltd, Paisley, UK). The Library of Pharmacologically-Active Compounds (LOPAC) containing 1280 compounds and all other chemical compounds were purchased from Sigma-Aldrich (St. Louis, MO, USA), unless indicated otherwise. The LOPAC contains marketed and candidates drugs as well as "gold standards" that have well-characterized activities. Each compound in the library was supplied as 10mM stock solution in DMSO and was screened at a final concentration of 1µM. The 804G cell line (kindly provided by Dr T. Otonkoski, University of Helsinki, Finland) and the method for preparation and application of its matrix have been described before (Bosco et al., 2000). Isolated beta cells were seeded in 804G matrix-coated, imaging-grade black 384 well plates (Greiner Bio-One, Frickenhausen, Germany) or, when indicated, in 6-well cell culture plates (BD Biosciences, San Jose, CA, USA).

**Purification, culture and analysis of rat beta cells:** All animal experiments were approved by the Ethical Committee for Animal Experimentation of the Vrije Universiteit Brussel and all manipulations were carried out in accordance with the European Community Council Directive 86/609/EEC. Experimental procedures for isolation of beta cells from neonatal (2-3 days old) and adult (8 or 40 week old, male) Wistar rats (Janvier Bioservices, France) and analysis of their number, viability and proliferation activity in culture have been described before (Assefa et al., 2014). Sorted beta cells were seeded in 804G matrix-coated plates (designated as day 0) and the numbers and viability of seeded cells at the start of all experiments were determined on day 1 (24h after seeding). The cells were cultured in control medium, which is a serum-free Ham's F10 medium supplemented with 2mM L-glutamine, 50µM 3-isobutyl-1-methylxanthine, 0.5% Albumax I, penicillin (100U/ml), streptomycin (0.1 mg/ml) and, unless indicated otherwise, 10mM glucose.

**Purification and culture of human beta cells:** Human islets preparations (average age of donors 43 years; range 14-68 years) were provided by the Beta Cell Bank of Brussels University Hospital (UZ Brussel, Brussels, Belgium), which obtains donor pancreas via its affiliation with the Eurotransplant Foundation (Leiden, The Netherlands). Single cell suspensions were obtained after collagenase, trypsin and DNase digestion, followed by FACS-purification to >75% insulin-positive cells based on staining with the zinc-binding fluorochrome 6-methoxy-(8-p-toluenesulfonamido)quinoline (Invitrogen). Isolated human beta cells were briefly re-aggregated prior to seeding in collagen IV-coated 384-well plates in serum-free OptiMEM supplemented with 7.5mM glucose.

**Drug screening and data analysis:** Freshly purified beta cells were cultured in control medium for 24h prior to the addition of the LOPAC test compounds (day 1). The culture medium was refreshed on day 3 and the cells were then cultured further with 5-ethynyl-2'-deoxyuridine (EdU; 10µM) up to day 6. Cell viability and percentage EdU incorporation were quantified after imaging and image analysis using the BD Pathway Bioimager, as described before (Assefa et al., 2014). To test the significance of compound effect on beta cell proliferation, average Z-score for each compound was calculated and a cut-off for potential positive hits was set at a Z-score of greater than three standard deviations. Thus, a compound is considered a hit only if it increases the percent EdU incorporation to above three standard deviation of the plate-averaged percent EdU incorporation..

**Statistical analysis:** Data are expressed as means ± standard error of the mean (SEM) of n independent experiments. Statistical significance of differences among means was assessed with Student's t-test or ANOVA with Tukey's test for multiple comparisons using GraphPad Prism (GraphPad Software, San Diego, USA). Differences were considered significant at *p* < 0.05.

### RESULTS

### High-content drug screening assay identifies glucocorticoids as inducers of beta cell proliferation.

A library of 1280 compounds (LOPAC, Sigma) was screened for drugs that recruit adult rat beta cells into proliferative activity by using cell survival and de novo DNA synthesis as primary criteria. Preliminary experiments in control medium have demonstrated that beta cells that incorporate EdU indeed undergo productive mitosis to form surviving, insulin-positive daughter cells (Data not shown). After testing the compounds at the concentration of 1µM, we identified 17 (1.3% of total) structurally and mechanistically diverse compounds as potential inducers of beta cell proliferation based on their Z-scores . This group of compounds include (±)-Bay K 8644, an L-type calcium channel agonist, that has previously been reported to induce beta cell proliferation. Remarkably, six different glucocorticoids (GCs) are among the 17 selected bioactive compounds, including those with the top four Z-score values (Table 5). These GCs are the most potent inducers of proliferation, increasing the percent EdU incorporating beta cells by 2.6- to 8.6-fold higher over control condition. The peak effects are induced by corticoids that have highest affinity for GC receptor rather than mineralocorticoid receptor. We also identified other 106 compounds (8.3% of total) that block baseline beta cell proliferation (reducing basal EdU incorporation by at least 50%; data not shown) and 11 compounds that showed acute to chronic beta cell toxicity during culture (Data not shown). Both groups of compounds will not be considered any further in this study. Overall, our data demonstrate that the assay can identify compounds with deleterious effects on beta cells and, importantly, established GCs a novel class of drugs that can induce in vitro rat beta cell proliferation.

**Table 5: Glucocorticoids recruit adult rat beta cells into DNA synthesis.**

| | **Relative Potencies** | | **Z-Score** | **Percent EdU⁺ cells** | **Percent viable cells** |
|---|---|---|---|---|---|
| Glucocorticoid | GR | MR | | | |
| Triamcinolone | 5 | +/- | 5.9 | 31.1 | 99 |
| Beclomethasone | 20 | +/- | 5.9 | 20.6 | 96 |
| Betamethasone | 25 | +/- | 5.5 | 21.1 | 95 |
| Hydrocortisone | 1 | 1 | 5.4 | 28.0 | 97 |
| Budesonide | | | 4.1 | 24.9 | 97 |
| Cortexolone | | | 4.1 | 13.1 | 97 |
| Control medium | - | - | - | 5.6 | 97 |

| | | | | | |
|---|---|---|---|---|---|
| Six out of the nine tested glucocorticoids have Z-scores above the cut-off limit of three standard deviations and could induce up to 8.6-fold more percent EdU⁺ cells than the control condition. The largest effects are observed with compounds with relatively higher potency for the glucocorticoid receptor (GR) than the mineralocorticoid receptor (MR). | | | | | |

### GCs increase the number of beta cells through time-dependent recruitment of resting cells into the cell cycle, an effect not observed in other cell types.

Induction of beta cell proliferation by in vitro and in vivo GC treatments has been reported before, but it was not clear if this results in increased cell numbers. Thus, two of the most widely used GCs, hydrocortisone (HC), a naturally-occurring GC, and its synthetic analog, 6-methylprednisolone (MP), were selected, and used alternatively, for further analysis of their effects on the long-term survival and proliferation of beta cells. Dose-response studies showed that both compounds show optimal potency at 1µM final concentration (data not shown). To evaluate the temporal aspect of proliferation, beta cells cultured with or without GCs were exposed to EdU in successive time frames during a two-week incubation period (Fig. 5A). Our data show that GC-induced EdU incorporation rate ranges from 2% per day between day 1 to 3 to 9-11% per day between day 6 and 9. The time-dependent increase in proliferation rate indicates the existence of heterogeneity in the proliferative response of individual beta cells to GCs.

In terms of changes in cell numbers, both HC and MP increased the absolute number of living beta cells in culture (Fig. 5B), leading to near population doubling in two-weeks. For both compounds, the formation of new cells followed a two-phase pattern: a slow but steady and significant increase in the number of living cells between day 1 and 9, followed by a sharp steep increase between day 9 and 15. GC-induced formation of new beta cells seems to be mediated by time-dependent recruitment of non-divided cells into the cell cycle, rather than repetitive cell cycle entry by a limited pool of cells (Data not shown). End-point cell viability remained very high both in control and in the presence of GCs (data not shown), suggesting that the increase in cell numbers was not due to improved cell survival alone. At the end of culture, there was no change in the purity of insulin-positive cells as confirmed by immunocytochemical analysis (data not shown).

We next tested cell type-specificity of HC-induced proliferation by using dissociated rat islet cells containing 15-20% alpha cells and 10-15% other cell types. After a 15-day culture, alpha cell numbers drastically decreased to 40 ± 3% and 57 ± 1% of the starting level in control and HC-containing media, respectively. Non-endocrine pancreatic cells were virtually undetectable on day 15, presumably died out due to the serum-free culture conditions. A separate set of experiments also showed that HC does not induce the proliferation of INS1 832/13 cell line in serum-free culture medium (data not shown). Altogether, these data suggest that the proliferation-inducing effect of GCs is mediated via pathways specific to primary beta cells.

Interestingly, GCs can also induce robust DNA synthesis in primary human beta cells (Fig. 5C) isolated from pancreas donors of a wide range of ages (17-68 years). GC treatment doubles the percent EdU-incorporating beta cells as compared to control condition, while the percent proliferation in non-beta cells remained identical (data not shown). Whether this increase in DNA synthesis eventually results in more beta cells is not clear but the results provide a proofof-principle for beta cell-specific induction of proliferation and the predictive value of our drug screening assay.

### GCs time-dependently recruit increasing percent of beta cells into DNA synthesis.

To evaluate the temporal aspect of proliferation, beta cells cultured with or without GCs were exposed to EdU in successive timeframes during a two-week incubation period (Fig. 6). EdU incorporation rate ranges from 2% per day between from day 1 to 3 to a high of 9-11% per day between day 6 and 9. GC-induced proliferation rates were significantly higher than control at almost all time frames. A sequential labeling experiment with two thymidine analogues (Teta et al., 2005) indicated that GC-induced formation of new beta cells was mediated by recruitment of non-divided cells into the cell cycle, rather than the induction of repeated cell cycle in a select subset of cells (Fig. 6). Thus, in line with these previous studies, beta cell proliferative response does not involve repetitive entry into the cell cycle by a selected pool of cells.

### Glucocorticoid, not mineralicorticoid, receptor mediates HC-induced increase in beta cell number.

Since HC is known to bind and potently activate both GC receptor (GR) and mineralocorticoid receptor (MR), it was necessary to establish the relative contribution of signaling pathways downstream of the two receptors. We, therefore, analyzed the effects of aldosterone, a specific MR agonist, spironolactone, a potent MR antagonist and mifepristone (RU486), a high affinity GR antagonist, on HC-induced rat beta cell proliferation. Our data suggest that the activation of MR by aldosterone does not increase rat beta cell numbers while spironolactone does not block HC-induced proliferation during a 15-day culture (Table 6), implying that the proliferation signal is relayed mainly through the GR signaling pathways. This conclusion was strengthened by the observation that RU486 blocked HC-induced rat beta cell proliferation both at the level of EdU incorporation (data not shown) and the number of living cells on day 15 (Table 6). It is noteworthy that both spironolactone and RU486 did not influence the basal level of beta cell proliferation.

**Table 6: Glucocorticoid, not mineralocorticoid, receptor activation is involved in HC-induced beta cell proliferation.**

| Treatment | Number of Living Cells on Day 15 (% of Day 1) |
|---|---|
| Control | 101±7 |
| Control + RU486 (1µM) | 104±5 |
| Control + Spironolactone (1µM) | 105±5 |
| Control + Aldosterone (0.1 µM) | 112±5 |
| HC | 156±4*** |
| HC + RU486 (1µM) | 110±5^{§} |
| HC + Spironolactone (1µM) | 154±3*** |

| | |
|---|---|
| Purified rat beta cells were seeded in control medium and treated as indicated for 15 days. The number of living cells on Day 15 is expressed as percent of cell numbers on Day 1 (means ± SEM, n= 3-8); compared to control: ***, p<0.001. | |

### Transient presence of HC is sufficient to generate its proliferation-inducing effects on beta cells and prevents their degranulation.

Continuous incubation of rat beta cells with HC leads to a 56% loss of insulin content on day 9 and more than 70% loss on day 15 (Table 7). It is not clear whether this was the characteristics of newly formed beta cells, indicates HC effect on insulin storage or associated with HC-induced down regulation of genes involved in insulin expression and synthesis. We, therefore, performed a switch experiment whereby HC was withdrawn from medium after 9 days and cells were kept in control medium for the last 6-days of culture. Analysis of cell numbers on day 15 indicated that beta cells continued to proliferate even after the removal of HC (Table 7), indicating that relatively transient pre-incubation with the drug was sufficient to recruit cells into proliferative phenotype. Moreover, the insulin content of the newly-formed cells on day 15 was fully restored to start levels (Table 7), despite the significant level of cell expansion. Similar results were obtained when the cells were treated with MP (data not shown). Interestingly, removal of the GCs also normalized the expression of insulin gene and other signature markers of mature beta cells (Fig. 7) as well as glucose-stimulated insulin secretion (Table 8).

**Table 7: Effect of transient treatment with HC on cellular insulin content and cell numbers.**

| | **Insulin Content (ng/10³ cells)** | | **Number of Living Cells on** |
|---|---|---|---|
| **culture condition** | **Day 9** | **Day 15** | **Day 15 (% of Day 1)** |
| 15 days, control | 23.7±0.9 | 35.3±3.6 | 102.5±6.1 |
| 15 days, HC | 14.0±1.4** | 9.3±0.7*** | 173.9±7.1*** |
| 9 days, HC; 6 days, control | - | 33.1 ±2.9^{§} | 168.1±6.8*** |

| | | | |
|---|---|---|---|
| Beta cells were cultured for 9 or 15 days in control medium or with HC. in one series of experiments, HC was removed from the culture on Day 9. The number of living cells was determined on Day 15 and represented as percentage of the number of cells on Day 1 (means ± SEM; n=4). Values of cellular insulin content represent means ± SEM of 4-9 independent experiments; **, p<0.01; ***, p<0.001 versus corresponding control medium; §, p<0.001 versus 15-day HC condition. | | | |

**Table 8: Preservation of in vitro beta cell functions after expansion.**

| **Culture Condition** | **Insulin Release (ng/10³ cells/h)** | **glucose-induced insulin secretion, Day 15 (% of content/h)** | | | |
|---|---|---|---|---|---|
| | | **2.5mM** | **5mM** | **10mM** | **20mM** |
| 15 days, basal | 1.7±0.1 | 20.4±2.1 | 25.9±2.3 | 33.1±2.6 | 40.3±2.8 |
| 15 days, 6MP | 0.9±0.1*** | 35.3±7.4 | 37.7±4.0 | 43.2±6.8 | 44.0±4.8 |
| 9 days, 6MP followed by 6 days, basal | 1.6±1 | 24.4±3.3 | 34.3±3.9 | 46.8±7.7 | 52.1±1.8* |

| | | | | | |
|---|---|---|---|---|---|
| Beta cells were cultured in basal conditions or in the presence of 6MP. The GC was kept continuously for 15 days or withdrawn on day 9 and cells were cultured in basal condition for the last 6-days of culture. The amount of insulin released per hour during the last 72h of culture per 10³ beta cells is shown. Values represent means ± SEM; ***, p<0.001 versus basal medium; n=3-5. A 120-minute in vitro static glucose-stimulated insulin release assay was conducted on day 15 cells after culture at different glucose concentrations in basal medium. The amount of released insulin is expressed as percentage of cellular content, per 10³ beta cells and the values are means ± SEM; *, p<0.05 versus basal control cells, n=3. | | | | | |

### HC-induced proliferation of beta cells is associated with elevated rates of glucose utilization and oxidation and is more pronounced in cells with higher metabolic responsiveness to glucose.

Because GC signaling controls metabolic processes including glucose metabolism and we have previously reported that glucose regulates rat beta cell numbers through effects on beta cell survival and proliferation (Assefa et al., 2014)( Example 1), we investigated whether HC-induced beta cell proliferation is associated with a change in glucose metabolism. First, the metabolic effect of a 6-day treatment with HC was analyzed by 2h glucose utilization and oxidation rates at different glucose concentrations. As shown in Figure 9, glucose utilization in control and HC-treated beta cells increased in a linear, dose-dependent manner between 2.5 and 20mM glucose. Glucose oxidation also showed a linear curve between 2.5 and 10mM glucose and leveled-off between 10 and 20mM concentrations. Both glucose utilization and oxidation levels at 10 and 20mM glucose were significantly higher in HC-treated cells than controls (p<0.05), suggesting an increased metabolic activity in proliferating beta cells. Further, HC treatment doubles the rates of both utilization and oxidation at 10mM glucose as compared to those in control cells (p<0.05). At 20mM glucose, HC-treated cells showed 50% more of both metabolic activities than control cells, although HC pretreatment led to relatively more non-oxidative utilization of glucose. The results suggest that the increase in glucose metabolism precedes the induction of considerable beta cell proliferation.

To analyze a possible causative association between glucose metabolism and GC-induced proliferation, purified beta cells were sorted according to their metabolic responsiveness to glucose into low- and high-responsive cells according to their level of NAD(P)H (Kiekens et al., 1992). After a 15-day culture, while all cell groups showed equally high levels of survival (data not shown) and baseline proliferative activities were not significantly different, HC-induced proliferation was significantly higher in beta cells with high metabolic responsiveness to glucose as compared to the less-responsive subpopulation (Table 9). Nevertheless, cells with the highest metabolic responsiveness to glucose did not proliferate significantly higher than the mixed cell population suggesting a distinct mechanism of action for GCs and additional levels of control in beta cell proliferation.

Beta cell heterogeneity in metabolic responsiveness to glucose is associated with differences in glucose phosphorylation, a rate-limiting glycolytic step catalyzed by glucokinase (GK). Thus, we reasoned that interference with this key enzyme might influence the induction of proliferation by GCs. Initially, we observed that a competitive inhibition of GK by mannoheptulose reduces baseline and GC-induced proliferation of beta cells (data not shown), implying the mitogenic effects of glucose metabolism. As the effect of mannoheptulose might be due to cell starvation, we tested the effect of pharmacological activation of GK. The hypothesis is that if increased glucose metabolism enhances GC-induced beta cell proliferation, then a GK activator (GKA) should additively augment the level of proliferation. As illustrated in Figure 10, GKA and MP synergistically induce rat beta cell proliferation at 5 and 10mM glucose, leading to EdU incorporation rates equivalent to those induced by 20mM glucose. At 5mM glucose, MP and GKA increase DNA synthesis by 3.5-fold as compared to that induced by MP alone (p<0.01). The synergistic effects of GKA and MP are still observed at 10mM glucose, where GKA increases the MP-induced DNA synthesis by 2.4-fold (p<0.05), but are lost at 20mM glucose. After 15-day culture, combination of 5mM glucose, MP and GKA leads to the formation of 159±6% more cells as compared to day 1 numbers, which is significantly (p<0.001) higher than 10mM glucose alone (112±5%) and equivalent to the 10mM glucose and MP condition (152±5%). A 15-day continuous treatment of beta cells with GKA at 10mM glucose induces cytotoxicity and a net cell loss, irrespective of the presence or absence of MP (data not shown).

**Table 9: HC-induced proliferation of beta cells is associated with glucose responsiveness.**

| | Number of Living Cells on Day 15 (% of Day 1) | | |
|---|---|---|---|
| Beta Cell Population | Mixed beta cell population | Glucose 7.5 high- responsive cells | Glucose 7.5 lowresponsive cells |
| Culture condition | | | |
| Control | 110.7±5.0 | 99.0±1.5 | 94.0±9.2 |
| HC | 169.0±9.3*,§ | 182.0±4.9**,† | 117.3±5.2 |

| | | | |
|---|---|---|---|
| Purified adult rat beta cells were sorted according to their glucose responsiveness at the time of isolation. The different cell populations were cultured for 15 days with or without HC. The data are means ± SEM; n = 3; *, p<0.05; **, p<0.01, versus corresponding control condition; §, p<0.05, †, p<0.01 versus lowresponsive beta cells. | | | |

### Beta cells generated by in vitro proliferation can normalize glycemia in diabetic mice.

The ability to induce beta cell proliferation in culture could be implemented in the preparation of grafts for transplantation if the newly formed cells exhibit metabolically adequate functions. In this respect was it relevant to adjust culture conditions so that the expanded beta cell mass was not degranulated. We compared the in vivo function and hormone reserve of implants that were formed by equal numbers of beta cells collected from either control or HC-treated preparations, HC being withdrawn for the last 6 days (as in **Table 7).** At the end of 15 days culture, cell yield after HC was 54 to 110 percent higher than in control (paired cultures from three different islet isolation experiments), thus allowing preparation of more grafts per experiment. Respective grafts contained a similar number of beta cells and a similar total hormone content (for the three experiments between 10 to 15µg insulin per graft). When implanted in the abdominal fat pad of alloxan-diabetic mice, both groups corrected hyperglycemia within one week and remained normalized over the four-week follow-up (**results not shown**) with stable circulating C-peptide levels (at week 1 and at week 5 post-transplantation, respectively: 2.3±0.2nM and 2.6±0.2nM in control and 2.4±0.2nM at 2.6±0.4nM in HC group). Both also presented an equally rapid normalization of glycemia following an intraperitoneal glucose load with similar kinetics to normal control animals **(****Fig. 8****).** Removal of the graft-bearing tissue at post-transplantation week 4 was rapidly followed by a reversal to hyperglycemia, indicating that the implants had controlled glycemia. Measurements of the insulin content in implants and pancreatic organs indicated that both groups had generated implants with comparable insulin reserve, representing approximately 50 percent of the content in the initial grafts, and exceeding the corresponding pancreatic insulin content by almost ten-fold **(Table 10).** These data demonstrate that the HC-amplified beta cell mass generates beta cell grafts that, on a cell number basis, are equally potent as control preparations.

**Table 10: Comparative insulin content of graft and pancreas from diabetic control, graftrecipient and normal control mice.**

| | Insulin Content (µg/sample) | | | |
|---|---|---|---|---|
| | control animals | | animals transplanted with cells from: | |
| Sample | diabetic (n=3) | normal (n=3) | control condition (n=5) | proliferation condition (n=10) |
| Graft | - | - | 6.9±1.9 | 7.2±1.3 |
| Pancreas | 0.1±0.0 | 28.5±4.1 | 0.3±0.2 | 0.7±0.2 |

| | | | | |
|---|---|---|---|---|
| Grafts and pancreata of diabetic control graft recipient and normal control animals were collected to quantify insulin content by RIA. The values represent microgram insulin per sample (means ± SEM). | | | | |

### EXAMPLE 3: GLUCOCORTICOID RECEPTOR TRANSACTIVATION STIMULATES ADULT RAT BETA CELLS TO RECAPITULATE A PROLIFERATIVE PHENOTYPE

The current study was undertaken to evaluate the role of the GR behind GC-stimulation of beta cell proliferation, and to dissociate between transrepression and transactivation. Glucocorticoids such as dexamethasone, interestingly displays both transactivation and transrepressive properties in a cell specific manner. Using a selective GR modulator, compound A (CPA) (De Bosscher et al., 2005) we show that GC's stimulate beta cells to recapitulate a phenotype similar to the phenotype observed in models with a physiological adaptation of the beta cell mass, such as during pregnancy, neonatal growth and obesity. Whereas this phenotypic adaptation points to involvement of both transactivation and transrepression mechanisms, our results with CPA further indicate that stimulation of beta cell replication solely relies on GR-transactivation and does not involve transrepression. Our findings suggest that variations in cortisol levels and local action could be part of the missing link(s) between metabolic demand and compensatory beta cell proliferation, and further point to the importance of GR-transactivation, an aspect which often has been overlooked.

### EXPERIMENTAL PROCEDURES

### Animal experiments and isolation of primary rat beta cells

Neonatal and young adult Wistar rats (8-10 wks, male, pregnant female) and Zucker fatty rats (10 wks) were used to purify primary beta cells. Briefly, pancreata were dissected and collagenase (0.3 mg/ml) digested. Endocrine islets were enriched by percol gradient (neonates) or handpicked after elutriation from the more than 100 µm-fraction under a dissection microscope (adult rats). The islets were then dissociated into 50-60% single-cell suspension in a calcium-free medium containing trypsin (Boehringer Mannheim; 5µg/ml), and DNase (Boehringer Mannheim; 2 µg/ml); and sorted into single beta-cells by autofluorescence-activated cell sorting (FACS Star, Beckton Dickinson, CA) using cellular light-scatter and FADautofluorescence as discriminating parameter (Van De Winkel and Pipeleers, 1983). For in vivo-studies with GCs young male Wistar rats received a single intraperitoneal injection with 1 mg/kg BW dexamethasone (water soluble, Sigma), daily at the onset of the dark cycle for 5 consecutive days. Control rats were given vehicle (saline). Animals (n=9) were weighed at start and on day 6, blood glucose levels were monitored. An oral glucose tolerance test was performed on day 5 on 3 animals from each group. After 6 days pancreases were dissected and used for IHC (n=4), or beta cells were purified as described (n=3). Animals were reared and bred according to Belgian regulations of animal welfare, study protocols were in accordance with the European Community Council Directive (86/609/EEC) and approved by the local ethical committee for Animal Experimentation.

### In vitro stimulation of beta cell proliferation

Sorted beta cells were seeded in 384-well or 6-well plates coated with 804G matrix, and kept in culture for up to 15 days at 37°C 5% CO₂ using Ham F10 supplemented with 1 mM L-glutamine, 0.1 mg/ml streptomycin, 0.075 mg/ml penicillin, 50 µM of isobutyl-1-methylxanthine (IBMX) at 10 mM D-glucose with and without synthetic GCs (6 methyl-prednisolone (6MP), dexamethasone (DEX), GR-modulators (compound A (CPA), mifepristone (MIF), and NFκB inhibitors (JSH-23 en Bay11-7085). Total cell number and beta cell viability was evaluated after incubation with Hoechst 33342 and propidium iodide. All chemicals were from Sigma Life Sciences or Merck. Quantification of cell numbers and analysis of beta-cell viability was done using the Pathway Bioimager (BD Biosciences) and using IPlab and Attovision software for background subtraction as described prior (Assefa et al., 2014). Replicating beta cells were quantified after 24 or 72 hrs incubation with 5-ethynyl-2'-deoxyuridine (EdU, a thymidine analog) using the ClickIT EDU Alexa Fluor assay (Invitrogen). EdU-positive cells were expressed as percentage of total cell number.

### Gene expression profiles of proliferation-stimulated beta cells

RNA was isolated from adult rat beta cells cultured for 1, 9 and 15 days in the absence or presence of 6MP and the gene expression profiles were obtained using Affymetrix rat Exon 1^{st} arrays and compared to the gene expression profile of freshly isolated rat beta cells (day 0). Changes in gene expression were considered significant with a cut of p-value below 0.0001, and further analyzed using the ingenuity pathway analysis platform and gene-set enrichment analysis software (GSEA)

### Quantitative PCR

RNA was extracted using RNeasy columns (Qiagen), and quality was verified using an Agilent Bioanalyzer. Following removal of genomic DNA (TURBO DNA-free, Ambion) and reversetranscription (High-Capacity cDNA Archive Kit, Applied Biosystems), gene targets were amplified on an ABI Prism 7700 using TaqMan Universal PCR Master Mix and TaqMan MGB probes (Applied Biosystems, assay IDs available on request). Expression levels of target genes were normalized to 4 housekeeping genes (β-actin, HPRT1, rplp2 and psmc5) (ΔCt) and expressed versus a control condition as calibrator (comparative ΔΔCt method).

### Immunochemistry and morphometrical analysis

Cells were fixed by adding a 20% formaldehyde solution to each well to make a final concentration of 3.7%. After fixing, the cells were washed with PBS-5% BSA, permeabilized using 0.5% triton X-100 and incubated at room temperature with primary antibodies. After this, the cells were washed with PBS-BSA and incubated with Allexa fluor secondary antibodies (invitrogen). For EdU detection, the Click-iT 488 reaction cocktail was prepared according to manufacturer's instructions and added to the cells for 30 min. Cells were counterstained with DAPI (Sigma) and analyzed using a Pathway Bioimager. Pancreas tissues were separated into head and tail portions to evaluate the expression of protein markers and to allow a morphometrical analysis as described prior (Chintinne et al., 2010). Each portion was inserted into a square-shaped holder for overnight fixation in 4% formaldehyde. After paraffin embedding 5 µm square sections were cut, deparaffinised and treated for antigen retrieval with citrate buffer (pH 6.0 at 99°C) followed by overnight incubation at room temperature with primary antibodies. Alexa Fluor-conjugated secondary antibodies were used and nuclei were stained with DAPI (Sigma-Aldrich) in fluorescent mounting medium (Dako). Primary antibodies were: guinea pig anti-insulin (kind gift C. Van Schravendijk, Belgium), mouse anti-glucagon, rabbit anti-Ki67 (Acris), mouse anti-Ki67 (Dako), rabbit anti-DLK1 (kind gift JH Nielssen, Denmark), goat anti-NPY, rabbit anti-NNAT (Sigma), rabbit anti-ICAM-1, rabbit anti-NR3C1 (Santa Cruz), rabbit anti-MafA (Bethyl) and rabbit anti-Pdx1 .

For the measurement of beta cell number and distribution over aggregates sections from the tail and head portions were systematically sampled at 250µm intervals to collect a total surface area corresponding to ~2% of total organ volume. Entire sections were stained, photographed (Pathway Bio-Imager 855 or 435) and analyzed (IPLab software; Becton Dickinson, San Jose, CA, USA) to obtain data on insulin-positive and insulin-negative surface areas, and on the number and localization of nuclei in the insulin-positive areas. By delineating individual insulin-positive cells, we ensured that insulin-positive areas did not include insulin-negative cells. Beta cell mass (milligram per pancreas) was calculated by multiplying relative insulin-positive area, as determined in sections, by pancreas weight. Beta cell number per section was determined by nuclear counting in insulin-positive areas and extrapolated to the whole organ on the basis of the fractional volume analyzed. Counted beta cell numbers were corrected by a factor of 2.3 to avoid overestimation as described (Chintinne et al., 2010). The average of individual beta cell volumes was calculated by dividing total beta cell volume as determined by the Cavalieri principle by total beta cell number.

### Statistical analysis

Data are expressed as means ± standard deviation or standard error of mean of the indicated number of independent experiments. Significant differences between experimental conditions were assessed using one-way anova with bonferroni post-test, or by two-way anova with bonferroni multiple comparison test, using Graphpad prism 4.00 and with an acceptance level of P < 0.05.

### RESULTS

### Segregation of glucocorticoid action reveals transactivation as mechanism behind stimulation of beta cell proliferation

Proliferation of adult rat and human beta cells can be stimulated under serum free culture conditions by glucocorticoid treatment (GC). Current study was undertaken to clarify the potential role of the glucocorticoid receptor (GR) in this action. Young adult rat beta cells were cultured in the presence of dexamethasone (DEX) or 6-alpha methylprednisolone (6MP) for 9 or up to 15 days, leading to increased DNA synthesis (day 9) and parallel expansion of their absolute number of living cells (day 15) **(****Figure 11 A-C).** In order to discriminate between GR transrepression or transactivation, and to explore potential down-stream mechanisms we used a GR modulator, compound A (CPA) (De Bosscher et al., 2005). CPA competes with endogenous glucocorticoids to bind to the GR ligand-binding domain and supports transrepression of NFkB (Robertson et al., 2010). Its specific allosteric interaction with the ligand-binding domain however prevents formation of the GR-dimers which are required for transactivation of GR-downstream genes (Robertson et al., 2010). CPA thus allows to dissociate between GR-dependent transrepression or transactivation (Rauch et al., 2011). The effect of culture in the presence of glucocorticoids with or without CPA, or the non-specific antagonist mifepristone (MIF) on day 15 beta cell numbers is shown in Figure 11. Data for EdU incorporation (day 6-9) are not shown. CPA on its own showed no effect on cell numbers **(****Figure 11 A)****,** nor viability (results not shown), but competed in a dose dependent way with DEX and 6MP to bind the ligand-binding domain and as such blocked agonist induced DNA-synthesis and beta cell expansion **(****Figure 11** **B,C).** Inhibition of proliferation was also obtained with antagonist MIF. Segregation of GR-action by CPA was further confirmed by looking at the mRNA level of known transrepression and transactivation target genes (ICAM1, CXCL11 versus FKBP5, MKP1), the results are summarized in **Figure 11** **D (or alternatively Table 11).** ICAM1 and CXCL11 mRNA levels were, as expected, suppressed by 6MP and CPA, an effect counteracted by MIF. FKBP5 and MKP1 were induced by 6MP, but suppressed when combined with CPA, which confirms competition between 6MP and CPA to bind GR. The mRNA levels of GR and FKBP4 were not changed. The effects of 6MP and CPA on M-phase cell cycle markers **(Table 11)** further confirm CPA-dependent suppression of glucocorticoid induced proliferation. Together these results support the interpretation that stimulation of beta cell proliferation requires transactivation of GR target genes, and does not involve NFkB transrepression. In further support, NFkB-inhibitors acting along different sites of the signaling cascade; JSH-23 (prevents nuclear translocation) and Bay 11-7085 (blocks IkB phosphorylation) showed no effect on beta cell proliferation, while suppressing ICAM1 mRNA levels **(****Figure 11 E-F).**

**Table 11: Segregation of glucocorticoid action; Effect of 6MP, CPA and MIF on mRNA levels of known GR trans-repression and trans-activation targets, cell cycle genes and beta cell phenotype markers. Changes in expression levels are expressed as ΔΔCt values relative to the basal vehicle control condition.**

| | **Basal control** | | | **6MP** | | |
|---|---|---|---|---|---|---|
| **Genes** | **Vehicle** | **MIF** | **CPA** | **Vehicle** | **MIF** | **CPA** |
| **Transrepression target genes** | | | | | | |
| ICAM1 | 1.0 ± 0.0 | **1.7. ± 0.5 ¹** | **0.3 ± 0.1 ³** | **0.1 ± 0.1 ³** | **0.7 ± 0.1 ^{c}** | **0.1 ± 0.0 ³** |
| CXCL11 | 1.0 ± 0.0 | 1.5 ± 0.6 | 1.1 ± 0.3 | **0.1 ± 0.0 ³** | **1.7 ± 0.3 ^{1,c}** | **0.1 ± 0.0 ³** |

| **Transactivation target genes** | | | | | | |
|---|---|---|---|---|---|---|
| FKBP5 | 1.0 ± 0.0 | 0.7 ± 0.2 | 1.3 ± 0.1 | **15.4 ± 2.2 ³** | **1.1 ± 0.1 ^{c}** | **8.7 ± 1.6 ^{3,c}** |
| MKP1 | 1.0 ± 0.0 | 1.3 ± 0.0 | 2.7 ± 1.1 | **20.7 ± 4.2 ³** | **1.4 ± 0.3 ^{c}** | **7.3 ± 1.7 ^{3,b}** |

| **Non-responsive genes** | | | | | | |
|---|---|---|---|---|---|---|
| NR3C1 | 1.0 ± 0.0 | 1.1 ± 0.1 | 1.0 ± 0.1 | 1.0 ± 0.1 | 1.0 ± 0.2 | 1.0 ± 0.2 |
| FKBP4 | 1.0 ± 0.0 | 0.7 ± 0.2 | 0.9 ± 0.2 | 1.2 ± 0.1 | 1.3 ± 0.1 | 0.9 ± 0.2 |

| **Cell cycle markers** | | | | | | |
|---|---|---|---|---|---|---|
| CyclinD1 | 1.0 ± 0.0 | 1.0 ± 0.1 | **0.7 ± 0.1 ¹** | 0.8 ± 0.1 | 1.2 ± 0.2 **^{a}** | **0.6 ± 0.1 ¹** |
| CDK4 | 1.0 ± 0.0 | 1.3 ± 0.2 | 1.1 ± 0.2 | **1.7 ± 0.2 ²** | 1.2 ± 0.2 | **1.7 ± 0.2 ²** |
| Cyclin E1 | 1.0 ± 0.0 | 0.7 .± 0.1 | 1.0 ± 0.1 | **2.3 ± 0.3³** | **0.8 ± 0.1 ^{c}** | **1.5 ± 0.1 ^{b}** |
| Cyclin B1 | 1.0 ± 0.0 | 1.0 ± 0.3 | 0.8 ± 0.4 | **4.7 ± 0.7 ³** | **1.1 ± 0.1 ^{c}** | **0.6 ± 0.2 ^{c}** |
| Cyclin B2 | 1.0 ± 0.0 | **0.6 ± 0.1 ¹** | **0.5 ± 0.1 ²** | **4.8 ± 0.8 ³** | **1.0 ± 0.2 ^{c}** | **0.6 ± 0.2 ^{c}** |

| **Selected phenotype markers** | | | | | | |
|---|---|---|---|---|---|---|
| DLK1 | 1.0 ± 0.0 | 0.9 ± 0.2 | 1.4 ± 0.4 | **24491 ± 5157 ³** | **86 ± 77 ^{c}** | **1400 ± 421 ^{c}** |
| NPY | 1.0 ± 0.0 | 0.8 ± 0.0 | 1.1 ± 0.3 | **3.8 ± 0.6 ³** | **1.1 ± 0.2 ^{c}** | **2.1 ± 0.5 ^{b}** |
| GDF11 | 1.0 ± 0.0 | 0.8 .± 0.2 | 0.7 ± 0.1 | 3.8 **± 1.0 ³** | **1.3 ± 0.5 ^{c}** | **1.9 ± 0.3 ^{b}** |
| TRH | 1.0 ± 0.0 | 0.6 ± 0.1 | 1.2 ± 0.6 | **5.3 ± 0.5 ³** | **1.8 ± 0.3 ^{c}** | **3.3 ± 0.7 ^{3,b}** |
| NNAT | 1.0 ± 0.0 | 0.8 .± 0.2 | 0.6 ± 0.1 | **7.4 ± 1.8 ³** | **2.0 ± 1.2 ^{b}** | **1.4 ± 0.2 ^{c}** |
| MafB | 1.0 ± 0.0 | 0.6 ± 0.2 | 1.4 ± 0.5 | **5.1 ± 0.9 ³** | **0.9 ± 0.2 ^{c}** | **2.7 ± 0.2 ^{b}** |
| MafA | 1.0 ± 0.0 | 1.1 ± 0.3 | **0.4 ± 0.1 ³** | **0.2 ± 0.1 ³** | **1.4 ± 0.4 ^{c}** | **0.1 ± 0.0 ³** |
| Pdx1 | 1.0 ± 0.0 | 1.1 ± 0.3 | 0.8 ± 0.1 | **0.5 ± 0.2 ³** | **1.3 ± 0.2 ^{c}** | **0.5 ± 0.1 ³** |

| | | | | | | |
|---|---|---|---|---|---|---|
| Beta cells were cultured for 9 days in basal control medium or medium enriched with 1µM 6MP, in absence (vehicle) or presence of 1 µM MIF, or 0.5 µM CPA. Ct-values were normalized to 4 house keeping genes (actin, hprt1, rplp2 and psmc5) and expressed relative to the basal vehicle control condition (ΔΔCt values) and analyzed by 2Way Anova with Bonferroni multiple comparison test, data are shown as mean ± SE, n = 4 - 6, ¹ p < 0.05, ² p < 0.01, ³ p < 0.001 as compared to Ctrl; ^{a} p < 0.05, ^{b} p < 0.01, ^{c} p < 0.001 as compared to 6MP+vehicle. | | | | | | |

### Glucocorticoid-stimulated beta cells recapitulate a phenotype with growth potential

Glucocorticoid-stimulation of beta cell proliferation required at least 72hrs incubation and was paralleled by a drastic change of phenotype as witnessed by phase contrast microscopy **(Data not Shown).** 6MP-treated beta cells show long thin branching cytoplasmic extrusions and dividing cells round-up and partially detach from the matrix (Data not shown).

In order to identify markers to describe these phenotype changes we analyzed the geneexpression profiles of beta cells after 1, 9 and 15 days 6MP-culture as compared to freshly isolated beta cells, and cells kept under control conditions (Affymetrix rat Exon 1st arrays,). As shown by principle component analysis (**Data not shown**), one day after isolation the profiles of control and 6MP cultured cells resembled each other and clustered together with fresh isolated cells, but after 9 and 15 days incubation the expression profiles of 6MP-incubated cells took a distant position, whereas the expression profiles of cells cultured without glucocorticoids resemble those of freshly isolated beta cells. This observation is consistent with prior work that showed preservation of beta cell function under the same conditions (Assefa 2014). 9-days 6MP treatment significantly altered the expression of 3011 genes (cutoff values: p < 0.0001 and fold change > 1.5). From this list we selected a number of genes for a more detailed expression-analysis based on their putative roles in beta cell differentiation, proliferation or function, and prior evidence regarding their glucocorticoid-dependent regulation, i.e. DLK1, NPY, NNAT, GDF11, TRH, MafA, MafB, PDX1 (**Data not shown**).

To this end, beta cells were cultured for up to 9 days with 6MP w/wo MIF or CPA, labeled 24hrs with EdU, and used for q-PCR or immunochemistry. Time course analysis showed that 6MP during the first 72 hrs even suppressed proliferation and that DNA-synthesis was only activated after prolonged exposure, a process that correlated in time with re-expression of DLK1 and NPY, and suppression of MafA (**Data not shown**). These observations suggest that glucocorticoids stimulate adult beta cells to recapitulate a phenotype with growth potential. We next questioned whether changes in the expression levels of these genes would reflect GR-dependent transactivation or -repression, and could be used as markers to document phenotype changes in glucocorticoid-activated beta cells. **Figure 12** **(or alternatively Table 11) and** **figure 13** summarize the q-PCR results and immunostainings. 6MP significantly increased the mRNA levels of DLK1, NPY, NNAT, TRH, GDF11 and MafB, all with a role during beta cell formation and maturation, changes that were counteracted by MIF and CPA. In parallel MafA and PDX1, two transcription factors considered characteristic for functionally mature beta cells, were suppressed by 6MP and by CPA which classifies these genes as transrepression-targets.

We further focused on DLK1, NPY, NNAT (transactivation), MafA, Pdx1, Icam1 (transrepression) and GR (unresponsive gene) to evaluate the expression of these genes at the protein level by ICC (data not shown). After 9 days 6MP-stimulated beta cells showed a strong but heterogeneous expression of DLK1 and NPY, and suppression of MafA and ICAM1. Comparable observations, but with less pronounced changes were made for NNAT (induced) and Pdx1 (suppressed) (results not shown). We observed no significant change in the expression of GR, which showed a nuclear signal in the majority of cells, and a strong cytoplasmic signal in a subtraction of beta cells (< 5%). Despite the correlation in time, we could not find a direct correlation between EdU-incorporation and the absence or presence of these makers.

These results underline that both transactivation and transrepression mechanisms are active in beta cells, and contribute to the phenotype-changes induced upon glucocorticoid-activation. Moreover, our results with CPA, which blocked 6MP-induced beta cell proliferation, clearly indicate that only the transactivation-dependent pathway is relevant to trigger beta cell proliferation.

### Glucocorticoid-stimulation of beta cell proliferation does not require paracrine signals

Dedifferentiation, as seems to occur after incubation with 6MP, can result in loss of sensitivity to growth-inhibiting signals and lead to production of own growth factors. Since DLK1, NPY, TRH and GDF11 are secretory factors, we next questioned whether beta cell proliferation could depend on the GR-induced production of these signals. Beta cells were cultured for 15 days in the presence of these molecules w/wo 6MP, the effect on cell numbers is shown in **Figure 14****.** Under basal conditions GDF11 and TRH showed a modest increase of cell numbers. In combination with 6MP TRH showed a further increase of cell numbers, whereas GDF11 suppressed proliferation. DLK1 showed no effect on cell numbers.. None of these factors equaled the increase induced by glucocorticoids. We did not test combinations of these molecules, but instead used preconditioned media to further explore this question. In prior work we already showed that beta cells continue to proliferate for more than 72hrs after removal of 6MP from the culture media and continue to express marker proteins. Medium was collected from 9 days 6MP-stimulated beta cells 72hrs after removal of 6MP, and used to culture fresh beta cells for 9 days. Cells were labeled with EdU between day 6 and 9, but we did not observe any stimulation of DNA synthesis (results not shown) which supports the conclusion that glucocorticoid-induced proliferation does not require paracrine factors.

### Glucocorticoid injection stimulates rat beta cells to recapitulate a phenotype with growth potential

Exogenous glucocorticoids have been reported to induce hyperinsulinemia, adaptive beta cell expansion followed by beta cell dysfunction resulting from an adaptive response to glucocorticoid induced insulin resistance and hyperglycemia (Rafacho et al., 2010). Our in vitro data however suggest a potential direct effect of glucocorticoids on beta cell replication via a GR-transactivation dependent mechanism. To explore this possibility we examined whether the phenotype changes observed in vitro could also be induced after intraperitoneal injection of dexamethasone. Young adult rats received daily injections (DEX, 1mg / Kg BW saline, or MOCK) for 5 consecutive days. Within this time frame the animals became mildly hyperglycemic and hyperinsulinemic, and lost 20% of their body weight (**Table 12**). Pancreasses were dissected 24hrs after the last injection and used for assessment of beta cell phenotype and morphometrical analysis of beta cell mass, or used for isolation of beta cells. Q-PCR analysis on isolated beta cells showed increased levels of transactivation targets (FKBP5, MKP1, DLK1, NPY, GDF11, TRH, NNAT, but not MafB) and suppression of transrepression targets (ICAM1, CXCL11, MafA, Pdx1) (**Table 13**). In parallel mRNA levels of key regulators of the M-phase of the cell cycle were induced. Immunohistochemical analysis showed a 3-fold increase of the percentage beta cells positive for Ki67, a heterogeneous expression of DLK1, and suppression of MafA and ICAM1 (**Data not shown**), which allows to conclude that beta cells in vivo indeed undergo the same phenotype changes in response to glucocorticoids.

**Table 12. Metabolic effects of in vivo dexamethasone administration**

| | | **Day 6** | |
|---|---|---|---|
| | | **MOCK** | **DEX** |
| Body weight (g) | Day 0 | 356 ± 8 | 363 ± 13 |
| | Day 6 | 382 ± 13 | 304 ± 11 * |
| Fasting glycemia (mg/dl) | | 85 ± 3 | 169 ± 77 *** |
| IPGT (mg/dl) | 15 min | 360 ± 43 | 497 ± 16 * |
| | 30 min | 180 ± 14 | 456 ± 41 *** |
| | 60 min | 83 ± 5 | 375 ± 54 *** |
| | 90 min | 81 ± 3 | 278 ± 44 *** |
| | 120 min | 82 ± 2 | 227 ± 62 *** |
| Serum insulin (pg/ml) | | 4927 ± 724 | 8447 ± 472 ** |
| Islet insulin content (ng/islet) | | 100 | 37 ** |
| insulin content (ng/K beta cells) | | 48 ± 7 | 22 ± 3 *** |

| | | | |
|---|---|---|---|
| Young adult rats received a daily injection (DEX, 1 mg / Kg BW saline, or MOCK) for 5 consecutive days. Pancreasses were dissected 24hrs after the last injection and used for isolation of islets and purification of beta cells, n= 3-6. | | | |

**Table 13: Effect of DEX injection on mRNA levels of selected gene targets in FACS-purified beta cells. Changes in expression levels are expressed as ΔΔCt values relative to the control condition (adult male)**

| **Genes** | **DEX** | **NEONATE** | **PREGNANCY** |
|---|---|---|---|
| **Transrepression target gene** | | | |
| ICAM1 | **0.1 ± 0.1 ³** | **0.2 ± 0.1 ³** | **0.3 ± 0.1 ³** |

| **Transactivation target gene** | | | |
|---|---|---|---|
| FKBP5 | **2.4 ± 0.2** | **2.7 ± 1.4¹** | 2.0 ± 0.5 |

| **Non responsive gene** | | | |
|---|---|---|---|
| NR3C1 | **0.5 ± 0.1 ²** | 1.7 ± 0.6 | 0.8 ± 0.3 |

| **Cell Cycle marker genes** | | | |
|---|---|---|---|
| CyclinD1 | 0.9 ± 0.4 | **0.2 ± 0.0 ³** | **0.3 ± 0.1 ³** |
| Cyclin E1 | **4.9.± 1.1 ³** | **4.7 ± 0.9 ³** | **5.4 ± 0.4 ³** |
| Cyclin B1 | **6.5 ± 2.5 ³** | **179.0 ± 17.9 ³** | **14.9 ± 6.8 ¹** |
| Cyclin B2 | **10.8 ± 5.1 ³** | **28.4 ± 9.7 ³** | **3.2 ± 0.2¹** |

| **Selected phenotype markers** | | | |
|---|---|---|---|
| DLK1 | **33.0 ± 19.1 ³** | **484.10³ ± 145.10^{3 3}** | **27.9 ± 2.6 ³** |
| NPY | **3.3 ± 0.5 ³** | **7.8 ± 5.63 ¹** | 3.1 ± 2.9 |
| GDF11 | 0.8 .± 0.2 | 0.9 ± 0.1 | 1.7 ± 0.9 |
| TRH | | **12.1 ± 9.0 ¹** | **3.7 ± 0.6 ¹** |
| NNAT | | **5.6 ± 0.0 ³** | **30.2 ± 3.0 ³** |
| MafB | 0.6 ± 0.2 | **438.4 ± 89.2 ³** | **110.2 ± 5.3 ³** |
| MafA | **0.3 ± 0.1** | **0.1 ± 0.0 ³** | **0.2 ± 0.1 ³** |
| Pdx1 | **0.4 ± 0.2** | **0.4 ± 0.2 ³** | **0.5 ± 0.1 ³** |

| | | | |
|---|---|---|---|
| Q-PCR analysis on FACS purified beta cells isolated from dexamethasone treated rats (DEX, 5 single injections DEX/saline, 1mg/kg BW daily), from neonates (day 2-3 after birth) or pregnant dams (E15). Ct-values normalized to 4 house keeping genes and expressed relative to the control condition (adult male), data are shown as mean ± se, n = 3-4, ¹ p < 0.05, ² p < 0.01, ³ p < 0.001. | | | |

### Glucocorticoids induce phenotype changes similar to those in proliferating beta cells under different physiological conditions

In order to examine whether the phenotype changes induced by glucocorticoids in vitro and in vivo would reflect a more general activation state of beta cells, we compared the expression profile of 9-day 6MP exposed cells to those of beta cells isolated from neonatal rats, pregnant rats (E15) and Zucker fatty rats (young adult), three models characterized by adaptive beta cell proliferation (**Table 13**). All four models showed a striking similarity of their gene expression profiles as shown by principle component analysis and heat-mapping (**Data not shown**). 113 genes were up-or down-regulated in all 4 models and showed, as expected, an enrichment of cell cycle regulators (33%), including GDF11, TRH, NNAT, ICAM1, CXCL11; all GR-targets.

Six genes that were down regulated in all models belonged to the I-kappaB kinase/NF-kappaB cascade (GO-functional classification) suggesting an overall suppression of NfkB-signaling during beta cell proliferation in all models.

To better understand these profiles, a comparison analysis was performed with Ingenuity Pathway Analysis to identify which canonical signaling pathways were enriched across the different datasets. As a negative control we also included the microarray profile of beta cells isolated after 48hrs fasting (which do not show proliferation) in this analysis. Out of 381 interrogated canonical signaling pathways, 34 pathways were found significantly (p-value < 0.05) enriched in at least 3 of the proliferation models (fisher's exact test right-tailed, cutoff criteria >1.5 fold change). Roughly these pathways could be subdivided in 4 groups; pathways involved in inflammation and stress responses, all with a central role for NfkB (14/34); nuclear receptor-signaling pathways (8/34); cell cycle control (5/34) and 7 miscellaneous. These observations are indicative for a central role of the GR receptor and glucocorticoid-signaling during beta cell expansion.

In order to evaluate this hypothesis we evaluated the expression of GR-target genes by Q-PCR and immunofluorescence, as compared to the changes induced after glucocorticoid injection in vivo. Q-PCR analysis indeed showed a comparable shift in neonatal beta cells and cells from pregnant rats with elevated levels of FKBP5, DUSP1, DLK1, NPY, TRH, NNAT; and suppression of ICAM1, CXCL11, MafA and Pdx1 (**Table 12**). Immunohistochemical staining showed elevated DLK1 expression in pancreas from neonatal and pregnant animals, and reduced expression of MafA and ICAM1 (**Data not shown**).

### References

Assefa, Z., Lavens, A., Steyaert, C., Stange, G., Martens, G.A., Ling, Z.D., Hellemans, K., and Pipeleers, D. (2014). Glucose Regulates Rat Beta Cell Number through Age-Dependent Effects on Beta Cell Survival and Proliferation. Plos One 9.
Bosco, D., Meda, P., Halban, P.A., and Rouiller, D.G. (2000). Importance of cell-matrix interactions in rat islet beta-cell secretion in vitro: role of alpha6beta1 integrin. Diabetes 49, 233-243.
Chintinne, M., Stange, G., Denys, B., In 't Veld, P., Hellemans, K., Pipeleers-Marichal, M., Ling, Z., and Pipeleers, D. (2010). Contribution of postnatally formed small beta cell aggregates to functional beta cell mass in adult rat pancreas. Diabetologia 53, 2380-2388.
De Bosscher, K., Vanden Berghe, W., Beck, I.M., Van Molle, W., Hennuyer, N., Hapgood, J., Libert, C., Staels, B., Louw, A., and Haegeman, G. (2005). A fully dissociated compound of plant origin for inflammatory gene repression. Proc Natl Acad Sci U S A 102, 15827-15832.
Heimberg, H., De Vos, A., Vandercammen, A., Van Schaftingen, E., Pipeleers, D., and Schuit, F. (1993). Heterogeneity in glucose sensitivity among pancreatic beta-cells is correlated to differences in glucose phosphorylation rather than glucose transport. EMBO J 12, 2873-2879.
Heit, J.J., Karnik, S.K., and Kim, S.K. (2006). Intrinsic regulators of pancreatic beta-cell proliferation. Annu Rev Cell Dev Biol 22, 311-338.
Hoorens, A., Van de Casteele, M., Kloppel, G., and Pipeleers, D. (1996). Glucose promotes survival of rat pancreatic beta cells by activating synthesis of proteins which suppress a constitutive apoptotic program. J Clin Invest 98, 1568-1574.
Kiekens, R., In 't Veld, P., Mahler, T., Schuit, F., Van De Winkel, M., and Pipeleers, D. (1992). Differences in glucose recognition by individual rat pancreatic B cells are associated with intercellular differences in glucose-induced biosynthetic activity. J Clin Invest 89, 117-125.
Ling, Z., Hannaert, J.C., and Pipeleers, D. (1994). Effect of nutrients, hormones and serum on survival of rat islet beta cells in culture. Diabetologia 37, 15-21.
Ling, Z., and Pipeleers, D.G. (1994). Preservation of glucose-responsive islet beta-cells during serum-free culture. Endocrinology 134, 2614-2621.
Nielsen, J.H., Svensson, C., Galsgaard, E.D., Moldrup, A., and Billestrup, N. (1999). Beta cell proliferation and growth factors. J Mol Med (Berl) 77, 62-66.
Pipeleers, D., Kiekens, R., Ling, Z., Wilikens, A., and Schuit, F. (1994). Physiologic relevance of heterogeneity in the pancreatic beta-cell population. Diabetologia 37 Suppl 2, S57-64. Pipeleers, D.G., in't Veld, P.A., Van de Winkel, M., Maes, E., Schuit, F.C., and Gepts, W.
(1985a). A new in vitro model for the study of pancreatic A and B cells. Endocrinology 117, 806-816.
Pipeleers, D.G., Schuit, F.C., Van Schravendijk, C.F., and Van de Winkel, M. (1985b). Interplay of nutrients and hormones in the regulation of glucagon release. Endocrinology 117, 817-823.
Rafacho, A., Quallio, S., Ribeiro, D.L., Taboga, S.R., Paula, F.M., Boschero, A.C., and Bosqueiro, J.R. (2010). The adaptive compensations in endocrine pancreas from glucocorticoid-treated rats are reversible after the interruption of treatment. Acta Physiol (Oxf) 200, 223-235.
Rauch, A., Gossye, V., Bracke, D., Gevaert, E., Jacques, P., Van Beneden, K., Vandooren, B., Rauner, M., Hofbauer, L.C., Haegeman, G., et al. (2011). An anti-inflammatory selective glucocorticoid receptor modulator preserves osteoblast differentiation. Faseb j 25, 1323-1332.
Robertson, S., Allie-Reid, F., Vanden Berghe, W., Visser, K., Binder, A., Africander, D., Vismer, M., De Bosscher, K., Hapgood, J., Haegeman, G., et al. (2010). Abrogation of glucocorticoid receptor dimerization correlates with dissociated glucocorticoid behavior of compound a. J Biol Chem 285, 8061-8075.
Ruhe, R.C., Curry, D.L., Herrmann, S., and McDonald, R.B. (1992). Age and gender effects on insulin secretion and glucose sensitivity of the endocrine pancreas. Am J Physiol 262, R671-676.
Salpeter, S.J., Klein, A.M., Huangfu, D., Grimsby, J., and Dor, Y. (2010). Glucose and aging control the quiescence period that follows pancreatic beta cell replication. In Development (England), pp. 3205-3213.
Salpeter, S.J., Klochendler, A., Weinberg-Corem, N., Porat, S., Granot, Z., Shapiro, A.M., Magnuson, M.A., Eden, A., Grimsby, J., Glaser, B., etal. (2011). Glucose regulates cyclin D2 expression in quiescent and replicating pancreatic beta-cells through glycolysis and calcium channels. In Endocrinology (United States), pp. 2589-2598.
Teta, M., Long, S.Y., Wartschow, L.M., Rankin, M.M., and Kushner, J.A. (2005). Very slow turnover of beta-cells in aged adult mice. In Diabetes (United States), pp. 2557-2567.
Teta, M., Rankin, M.M., Long, S.Y., Stein, G.M., and Kushner, J.A. (2007). Growth and regeneration of adult beta cells does not involve specialized progenitors. In Dev Cell (United States), pp. 817-826.
Van De Winkel, M., and Pipeleers, D. (1983). Autofluorescence-activated cell sorting of pancreatic islet cells: purification of insulin-containing B-cells according to glucose-induced changes in cellular redox state. Biochem Biophys Res Commun 114, 835-842.
Van de Winkle, M., Maes, E., and Pipeleers, D. (1982). Islet cell analysis and purification by light scatter and autofluorescence. Biochem Biophys Res Commun 107, 525-532.

## Claims

1. A method for identifying a compound that stimulates beta cell differentiation, proliferation and/or expansion, by determining the ability of said compound to induce the glucocorticoid receptor dependent-transactivation pathway in said beta cells, wherein a compound that is capable of inducing the glucocorticoid receptor-dependent transactivation pathway, is identified as a compound that stimulates beta cell differentiation, proliferation and/or expansion.

2. A method according to claim 1, wherein testing the ability of said compound to induce the glucocorticoid receptor dependent-transactivation pathway in said beta cells comprises the steps of:
- culturing beta cells or precursors thereof in the absence (control sample), or presence (test sample) of a test compound;
- measuring the expression of one or more transactivation markers in said beta cells or precursors thereof in both samples;
wherein a compound that increases expression of said one or more transactivation markers in said test sample in comparison with said control sample, is identified as a compound that stimulates beta cell differentiation, proliferation and/or expansion.

3. A method according to claim 2, wherein said one or more transactivation markers are selected from the list comprising: FKBP5, MKP1, IL-1Ra, Fgb and Fgg.

4. A method according to any one of claims 2 or 3 further comprising measuring the expression of one or more transrepression markers in said beta cells;
wherein a compound that increases expression of one or more transactivation markers, and does not decrease the expression of one or more transrepression markers in said test sample in comparison with said control sample; is identified as a compound that stimulates beta cell differentiation, proliferation and/or expansion.

5. A method according to claim 4, wherein said one or more transrepression markers are selected from the list comprising: ICAM1, CXCL11, Nfkbie, Myd88, Birc3.

6. A method for identifying a compound that stimulates beta cell differentiation over time, said method comprising the steps of:
- providing a compound that is capable of inducing the glucocorticoid receptor dependent-transactivation pathway in beta cells or precursors thereof;
- culturing beta cells or precursors thereof in the presence of said compound;
- measuring over time in said beta cells the expression of one or more differentiation markers, selected from MafB, DLK1, NPY, NNAT and TRH; and one or more maturation markers selected from MafA and PDX1;
wherein a compound that increases expression of at least one of said differentiation markers and does not decrease the expression of said one or more maturation markers, over time, is identified as a compound that stimulates beta cell differentiation.

7. A method for identifying a compound that stimulates beta cell proliferation over time, said method comprising the steps of:
- providing a compound that is capable of inducing the glucocorticoid receptor dependent-transactivation pathway in beta cells or precursors thereof;
- culturing beta cells or precursors thereof in the presence of said compound and a thymidine-analog;
- measuring the degree of incorporation of said thymidine-analog in said beta cells over time;
wherein a compound that increases thymidine incorporation in said beta cells over time, is identified as a compound that influences beta cell proliferation.

8. A method for identifying a compound that stimulates beta cell expansion over time, said method comprising the steps of:
- providing a compound that is capable of inducing the glucocorticoid receptor dependent-transactivation pathway in beta cells or precursors thereof;
- culturing beta cells or precursors thereof in the presence of said compound;
- measuring the absolute number of living beta cells over time using high content imaging;
wherein a compound that increases the absolute number of living beta cells over time is identified as a compound that stimulates beta cell expansion.

9. A method according to anyone of claims 6 to 8, wherein said compound that is capable of inducing the glucocorticoid receptor dependent-transactivation pathway, is identified using the method according to any one of claims 1 to 5.

10. A method for identifying a compound suitable for the treatment of diabetes mellitus; said method comprising the steps of
- performing a method according to any one of claims 1 to 5,
- performing a method according to claim 6,
- performing a method according to claim 7, and
- performing a method according to claim 8;
wherein a compound that is capable of inducing the glucocorticoid receptor dependent-transactivation pathway in beta cells or precursors thereof as determined in anyone of claims 1 to 5, is capable of stimulating beta cell differentiation as determined in claim 6, is capable of stimulating beta cell proliferation as determined in claim 7, and is capable of stimulating beta cell expansion as determined in claim 8; is identified as a compound that is suitable for the treatment of diabetes mellitus.

11. The method according to anyone of claims 1 to 10, wherein said beta cells or precursors thereof are cultured on extracellular matrices.

## Patentansprüche

1. Verfahren zum Identifizieren einer Verbindung, die Betazellen-Differenzierung, -Proliferation und/oder -Expansion stimuliert, durch Bestimmen der Fähigkeit der Verbindung, den Glukokortikoidrezeptor-abhängigen Transaktivierungsweg in den Betazellen zu induzieren, wobei eine Verbindung, die in der Lage ist, den Glukokortikoidrezeptor-abhängigen Transaktivierungsweg zu induzieren, als eine Verbindung identifiziert wird, die die Betazellen-Differenzierung, - Proliferation und/oder -Expansion stimuliert.

2. Verfahren nach Anspruch 1, wobei das Testen der Fähigkeit der Verbindung, den Glukokortikoidrezeptor-abhängigen Transaktivierungsweg in den Betazellen zu induzieren, die folgenden Schritte umfasst:
- Kultivieren von Betazellen oder Vorläufern davon in Abwesenheit (Kontrollprobe) oder Anwesenheit (Testprobe) einer Testverbindung;
- Messen der Expression eines oder mehrerer Transaktivierungsmarker(s) in den Betazellen oder Vorläufern davon in beiden Proben;
wobei eine Verbindung, die die Expression eines oder mehrerer Transaktivierungsmarker(s) in der Testprobe im Vergleich zur Kontrollprobe erhöht, als eine Verbindung identifiziert wird, die die Betazellen-Differenzierung, -Proliferation und/oder -Expansion stimuliert.

3. Verfahren nach Anspruch 2, wobei ein oder mehrere Transaktivierungsmarker aus der Liste ausgewählt sind, die Folgendes umfasst: FKBP5, MKP1, IL-1Ra, Fgb und Fgg.

4. Verfahren nach einem der Ansprüche 2 oder 3, ferner umfassend das Messen der Expression von einem oder mehreren Transrepressionsmarkern in den Betazellen;
wobei eine Verbindung, die die Expression von einem oder mehreren Transaktivierungsmarkern erhöht und die Expression von einem oder mehreren Transrepressionsmarkern in der Testprobe im Vergleich zur Kontrollprobe nicht verringert; wird als eine Verbindung identifiziert, die die Betazellen-Differenzierung, -Proliferation und/oder -Expansion stimuliert.

5. Verfahren nach Anspruch 4, wobei einer oder mehrere Transrepressionsmarkern aus der Liste ausgewählt sind, die umfasst: ICAM1, CXCL11, Nfkbie, Myd88, Birc3.

6. Verfahren zum Identifizieren einer Verbindung, die die Betazellen-Differenzierung im Laufe der Zeit stimuliert, wobei das Verfahren die folgenden Schritte umfasst:
- Bereitstellen einer Verbindung, die in der Lage ist, den Glukokortikoidrezeptor-abhängigen Transaktivierungsweg in Betazellen oder Vorläufern davon zu induzieren;
- Kultivieren von Betazellen oder Vorläufern davon in Anwesenheit der Verbindung;
- zeitliches Messen der Expression eines oder mehrerer Differenzierungsmarker(s), ausgewählt aus MafB, DLK1, NPY, NNAT und TRH, in den Betazellen; und einen oder mehrere Reifungsmarker, ausgewählt aus MafA und PDX1;
wobei eine Verbindung, die die Expression von mindestens einem der Differenzierungsmarker erhöht und die Expression eines oder mehrerer Reifungsmarker im Laufe der Zeit nicht verringert, als eine Verbindung identifiziert wird, die die Differenzierung von Betazellen stimuliert.

7. Verfahren zum Identifizieren einer Verbindung, die die Betazellen-Proliferation im Laufe der Zeit stimuliert, wobei das Verfahren die folgenden Schritte umfasst:
- Bereitstellen einer Verbindung, die in der Lage ist, den Glukokortikoidrezeptor-abhängigen Transaktivierungsweg in Betazellen oder Vorläufern davon zu induzieren;
- Kultivieren von Betazellen oder Vorläufern davon in Anwesenheit der Verbindung und eines Thymidin-Analogons;
- Messen des Einbaugrades des Thymidin-Analogons in die Betazellen über die Zeit;
wobei eine Verbindung, die den Thymidin-Einbau in die Betazellen über die Zeit erhöht, als eine Verbindung identifiziert wird, die die Betazellen-Proliferation beeinflusst.

8. Verfahren zum Identifizieren einer Verbindung, die die Betazellen-Expansion im Laufe der Zeit stimuliert, wobei das Verfahren die folgenden Schritte umfasst:
- Bereitstellen einer Verbindung, die in der Lage ist, den Glukokortikoidrezeptor-abhängigen Transaktivierungsweg in Betazellen oder Vorläufern davon zu induzieren;
- Kultivieren von Betazellen oder Vorläufern davon in Anwesenheit der Verbindung;
- Messen der absoluten Zahl lebender Betazellen im Laufe der Zeit unter Verwendung von High-Content-Imaging;
wobei eine Verbindung, die die absolute Anzahl lebender Betazellen im Laufe der Zeit erhöht, als eine Verbindung identifiziert wird, die die Betazellen-Expansion stimuliert.

9. Verfahren nach einem der Ansprüche 6 bis 8, wobei die Verbindung, die in der Lage ist, den Glukokortikoidrezeptor-abhängigen Transaktivierungsweg zu induzieren, unter Verwendung des Verfahrens nach einem der Ansprüche 1 bis 5 identifiziert wird.

10. Verfahren zur Identifizierung einer Verbindung, die zur Behandlung von Diabetes mellitus geeignet ist; wobei das Verfahren die folgenden Schritte umfasst:
- Durchführen eines Verfahrens nach einem der Ansprüche 1 bis 5,
- Durchführen eines Verfahrens nach Anspruch 6,
- Durchführen eines Verfahrens nach Anspruch 7 und
- Durchführen eines Verfahrens nach Anspruch 8;
wobei eine Verbindung, die den Glukokortikoidrezeptor-abhängigen Transaktivierungsweg in Betazellen oder Vorläufern davon induzieren kann, wie in einem der Ansprüche 1 bis 5 bestimmt, Betazellen-Differenzierung stimulieren kann, wie in Anspruch 6 bestimmt, Betazellen-Proliferation stimulieren kann, wie in Anspruch 7 bestimmt, und in der Lage ist, die Betazellen-Expansion zu stimulieren, wie in Anspruch 8 bestimmt; wird als eine Verbindung identifiziert, die zur Behandlung von Diabetes mellitus geeignet ist.

11. Verfahren nach einem der Ansprüche 1 bis 10, wobei die Betazellen oder Vorläufer davon auf extrazellulären Matrizen kultiviert werden.

## Revendications

1. Un procédé permettant d'identifier un composé qui stimule la différenciation, la prolifération et/ou l'expansion des cellules bêta, en déterminant la capacité dudit composé à induire la voie de transactivation dépendante du récepteur des glucocorticoïdes dans lesdites cellules bêta, dans lequel un composé capable d'induire la voie de transactivation dépendante du récepteur des glucocorticoïdes, est identifié comme un composé qui stimule la différenciation, la prolifération et/ou l'expansion des cellules bêta.

2. Un procédé selon la revendication 1, dans lequel le test de la capacité dudit composé à induire la voie de transactivation dépendante du récepteur des glucocorticoïdes dans ces cellules bêta comprenant les étapes suivantes :
- mettre en culture des cellules bêta ou leurs précurseurs en l'absence (échantillon témoin), ou en présence (échantillon pour essai) d'un composé test ;
- mesurer l'expression d'un ou plusieurs marqueurs de transactivation dans lesdites cellules bêta ou les précurseurs de ces dernières dans les deux échantillons ;
dans lequel un composé qui augmente l'expression dudit ou desdits marqueur(s) de transactivation dans ledit échantillon pour essai par rapport à l'échantillon témoin, est identifié comme un composé qui stimule la différenciation, la prolifération et/ou l'expansion des cellules bêta.

3. Un procédé selon la revendication 2, dans lequel un ou plusieurs marqueurs de transactivation sont sélectionnés dans la liste comprenant : FKBP5, MKP1, IL-1Ra, Fgb et Fgg.

4. Un procédé selon l'une quelconque des revendications 2 ou 3 comprenant la mesure de l'expression d'un ou plusieurs marqueurs de transrépression dans lesdites cellules bêta ;
dans lequel un composé qui augmente l'expression d'un ou de plusieurs marqueurs de transactivation, et ne diminue pas l'expression d'un ou de plusieurs marqueurs de transrépression dans ledit échantillon pour essai par rapport à l'échantillon témoin ; est identifié comme un composé qui stimule la différenciation, la prolifération et/ou l'expansion des cellules bêta.

5. Un procédé selon la revendication 4, dans lequel un ou plusieurs marqueurs de transrépression sont sélectionnés dans la liste comprenant : ICAM1, CXCL11, Nfkbie, Myd88, Birc3.

6. Un procédé d'identification d'un composé qui stimule la différenciation des cellules bêta dans le temps, ledit procédé comprenant les étapes suivantes :
- fournir un composé capable d'induire la voie de transactivation dépendante du récepteur des glucocorticoïdes dans les cellules bêta ou leurs précurseurs ;
- mettre en culture des cellules bêta ou leurs précurseurs en présence dudit composé ;
- mesurer, au fil du temps, dans lesdites cellules bêta l'expression d'un ou plusieurs marqueurs de différenciation, sélectionnés parmi MafB, DLK1, NPY, NNAT et TRH ; et un ou plusieurs marqueurs de maturation sélectionnés parmi MafA et PDX1 ;
dans lequel un composé qui augmente l'expression d'au moins un de ces marqueurs de différenciation et ne diminue pas l'expression de ce(s) marqueur(s) de maturation, au fil du temps, est identifié comme un composé qui stimule la différenciation des cellules bêta.

7. Un procédé d'identification d'un composé qui stimule la prolifération des cellules bêta dans le temps, ledit procédé comprenant les étapes suivantes :
- fournir un composé capable d'induire la voie de transactivation dépendante du récepteur des glucocorticoïdes dans les cellules bêta ou leurs précurseurs ;
- mettre en culture des cellules bêta ou leurs précurseurs en présence dudit composé et d'un analogue de la thymidine ;
- mesurer le degré d'incorporation dudit analogue de thymidine dans lesdites cellules bêta au fil du temps ;
dans lequel un composé qui augmente l'incorporation de thymidine dans lesdites cellules bêta au fil du temps, est identifié comme un composé qui influence la prolifération des cellules bêta.

8. Un procédé d'identification d'un composé qui stimule l'expansion des cellules bêta dans le temps, ledit procédé comprenant les étapes suivantes :
- fournir un composé capable d'induire la voie de transactivation dépendante du récepteur des glucocorticoïdes dans les cellules bêta ou leurs précurseurs ;
- mettre en culture des cellules bêta ou leurs précurseurs en présence dudit composé ;
- mesurer le nombre absolu de cellules bêta vivantes au fil du temps à l'aide d'une imagerie à haut contenu ;
dans lequel un composé qui augmente le nombre absolu de cellules bêta vivantes au fil du temps est identifié comme un composé qui stimule l'expansion des cellules bêta.

9. Un procédé selon l'une quelconque des revendications 6 à 8, dans lequel ledit composé capable d'induire la voie de transactivation dépendante du récepteur des glucocorticoïdes, est identifié à l'aide du procédé selon l'une quelconque des revendications 1 à 5.

10. Un procédé d'identification d'un composé adapté au traitement du diabète sucré ; ce procédé comprenant les étapes suivantes :
- mettre en oeuvre un procédé selon l'une quelconque des revendications 1 à 5 ;
- mettre en oeuvre un procédé selon la revendication 6,
- mettre en oeuvre un procédé selon la revendication 7, et
- mettre en oeuvre un procédé selon la revendication 8 ;
dans lequel un composé capable d'induire la voie de transactivation dépendante du récepteur des glucocorticoïdes dans les cellules bêta ou leurs précurseurs tels que déterminés dans l'une quelconque des revendications 1 à 5, est capable de stimuler la différenciation des cellules bêta telle que déterminée dans la revendication 6, est capable de stimuler la prolifération des cellules bêta telle que déterminée dans la revendication 7 et est capable de stimuler l'expansion des cellules bêta telle que déterminée dans la revendication 8 ; est identifié comme un composé adapté au traitement du diabète sucré.

11. Le procédé selon l'une quelconque des revendications 1 à 10, dans lequel lesdites cellules bêta ou leurs précurseurs sont mis en culture sur des matrices extracellulaires.
